# EUROPEAN PATENT SPECIFICATION

(11) **EP 0 313 873 B1**
(45) Date of publication and mention of the grant of the patent: **28.09.1994**
(21) Application number: 88116338.0
(22) Date of filing: 03.10.1988
(51) Int. Cl.: A61K 47/00, A61K 39/395, C07H 15/203

(54) **Targeted forms of methyltrithio antitumor agents**
Targetformer von Antitumor-Methyltrithioagenzien
Formes cibles d'agents méthyltrithio antitumoraux

(30) Priority: 30.10.1987 US 114940
(43) Date of publication of application: 03.05.1989
(73) Proprietor: AMERICAN CYANAMID COMPANY, Wayne, NJ 07470-8426 (US)
(72) Inventor: McGahren, William James, Demarest New Jersey 07627 (US); Sassiver, Martin Leon, Spring Valley New York 10977 (US); Ellestad, George A., Pearl River New York 10965 (US); Hamann, Philip R., Garnerville New York 10923 (US); Hinman, Lois M., North Tarrytown New York 10591 (US); Upeslacis, Janis, Pomona New York 10970 (US)
(74) Representative: Wächtershäuser, Günter, Prof. Dr.

(56) References cited:
- EP-A- 0 040 506
- EP-A- 0 132 082
- EP-A- 0 162 781
- FR-A- 2 430 943
- JOURNAL OF THE AMERICAN CHEMICAL SOCIETY, vol. 110, no. 14, 6th July 1988, pages 4866-4868, American Chemical Society; K.C.NICOLAOU et al.: "Cyclic conjugated enediynes related to calicheamicins and esperamicins: calculations, synthesis, and properties"

## Description

The invention describes carrier-drug conjugates of the disulfide analogs of the α₁ , α₂, α₃, α4, β₁ , β₂, λ₁, and 6 components of the LL-E33288 complex as well as the disulfide analogs of BBM-1675, FR-900405, FR-900406, PD 114759, PD 115028, CL-1577A, CL-1577B, CL-1577D, CL-1577E and CL-1724 antitumor antibiotics. The carrier portion of the conjugate is a mono- or polyclonal antibody, their fragments, chemically or genetically manipulated counterparts, growth factors or steroids. The invention includes compositions of the carrier-drug conjugates as well as their process of manufacture.

### DESCRIPTION OF THE DRAWINGS

Figure I: The ultraviolet spectrum of the antitumor antibiotic designated as LL-E33288_{Υ}1^{,}
Figure II: The proton magnetic resonance spectrum of the antitumor antibiotic designated as LL-E33288_{Υ1}, .
Figure III: The infrared spectrum of the antitumor antibiotic designated as LL-E33288_{Υ1},

The family of antibacterial and antitumor agents, known collectively as the LL-E33288 complex are described and claimed in copending U.S. patent application, Serial No. 009,321, filed January 30,1987 and are used to prepare the disulfur antitumor agents which are starting materials for targeted forms of the antitumor agents of our invention.

The Serial No. 009,321 application describes the LL-E33288 complex, the components thereof, namely, LL-E33288α₁, ^{Br}, LL-E33288α₁' , LL-E33288α₂ ^{Br}, LL-E33288α₂^{l} , LL-E33288α₃ ^{Br}, LL-E33288α₃^{l} , LL-E33288α₄ ^{Br}, LL-E33288β₁^{Br}, LL-E33288β₁^{l}, LL-E33288β₂^{Br}, LL-E33288β₂^{l} , LL-E33288_{Υ}1^{Br}, LL-E33288_{Υ}1 and LL-E33288δ₁, and methods for their production by aerobic fermentation utilizing a new strain of Micro- monospora echinospora ssp calichensis or natural or derived mutants thereof. Serial No. 009,321 also discloses proposed structures for some of the above named components. These proposed structures are reproduced in Table 1.

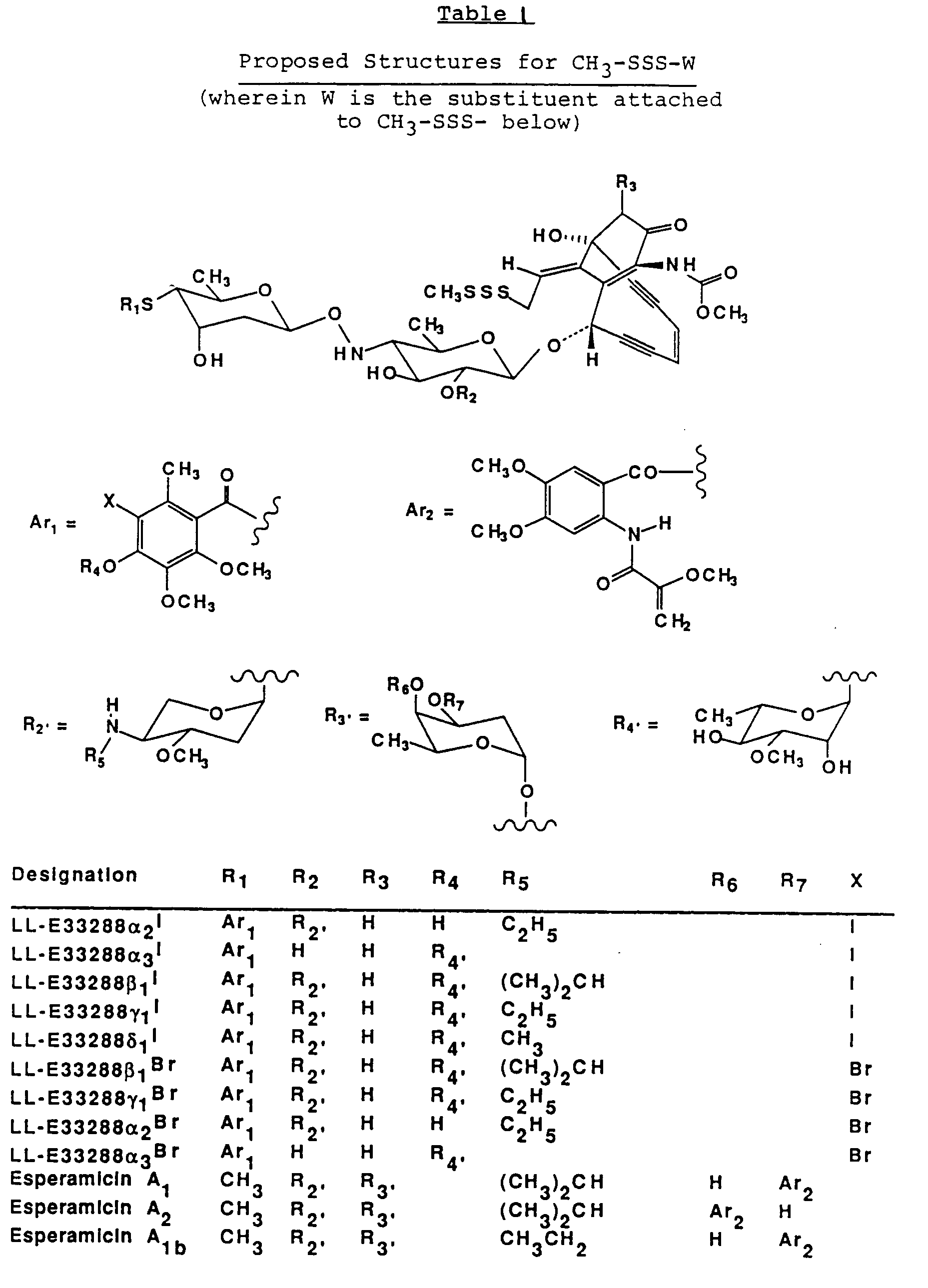

Certain other antibiotics are useful in our invention, namely:
1) Esperamicin BBM-1675, a novel class of potent antitumor antibiotics. I. Physico-chemical data and partial structure. M. Konishi, et. al., J. Antibiotics, 38, 1605 (1985). A new antitumor antibiotic complex, M. Konishi, et. al., U.K. Patent Application GB 2,141,425A, May 15, 1984.
2) New antitumor antibiotics, FR-900405 and FR-900406.1. Taxonomy of the producing strain. M. Iwami, et. al., J. Antibiotics 38, 835 (1985). New antitumor antibiotics FR-900405 and FR-900406.11. Production, isolation, characterization and antitumor activity. S. Kiyoto, et. al., J. Antibiotics, 38, 340 (1985).
3) PD 114759 and PD 115028, novel antitumor antibiotics with phenomenal potency. I. Isolation and characterization. R.H. Bunge, et. al., J. Antibiotics, 37, 1566 (1984). Biological and biochemical activities of the novel antitumor antibiotic PD 114759 and related derivatives. D.W. Fry et. al., Investigational New Drugs, 4, 3 (1986).
4) New antibiotic complex CL-1577A, CL-1577B produced by Streptomyces sp. ATCC 39363. European Patent Application 0,132,082, A2.
5) CL-1577D and CL-1577E Antibiotic antitumor compounds, their production and use. U.S. Patent 4,539,203.
6) CL-1724 Antibiotic compounds, their production and use. U.S. Patent 4,554,162.

The complete structures of esperamicins A₁, A₂, and A_{lb} (the BBM-1675 complex) have been reported, and these are included in Table 1. The physical characteristics of the above-named antitumor antibiotics indicate that they all are identical or very similar in structure to the esperamicins, and all contain a methyltrithio functional group.

As can be seen from the structures disclosed above, the α₁, α₂, α₃, α₄, β₁, β₂, γ₁ and 6 components of the LL-E33288 complex, as well as the BBM-1675, FR-900405, FR-900406, PD 114759, PD 115028, CL-1577A, CL-1577B, CL-1577D, CL-1577E and CL-1724 antibiotics each contain a methyltrithio group in their structure. The methyltrithio moiety of the above-named antibiotics is subject to displacement by a variety of thiol-containing organic molecules resulting in the formation of a new class of anticancer and antibacterial agents.

It has now been discovered that the displacement of the methyltrithio unit of the compounds listed in Table 1 and as depicted in Scheme I can be used to introduce a spacer (Sp), the judicious choice of which enable the introduction of targeting units into the compounds of the above-named patents and applications. 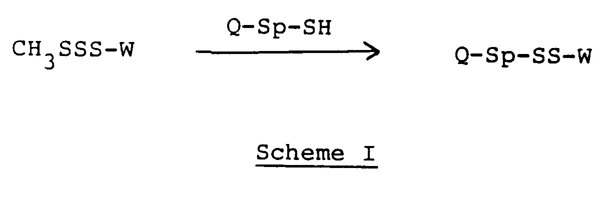 wherein Sp is a straight or branched-chain divalent (C₁-C₁₈) radical, divalent aryl or heteroaryl radicals, divalent (C₃-C₁₈) cycloalkyl or heterocycloalkyl radicals, divalent aryl- or heteroaryl-alkyl (C₁-C₁₈) radicals, divalent cycloalkyl- or heterocycloalkyl-alkyl (C₁-C₁₈) radicals, or divalent (C₂-Cₗ₈) unsaturated alkyl radicals; Q is, or can be subsequently converted to, halogen, amino, alkylamino, carboxyl, carboxaldehyde, hydroxy, thiol, a-haloacetyloxy, lower alkyldicarboxyl, -CONHNH₂, -NHCONHNH₂, -NHCSNHNH₂, -ONH₂, -CON3, 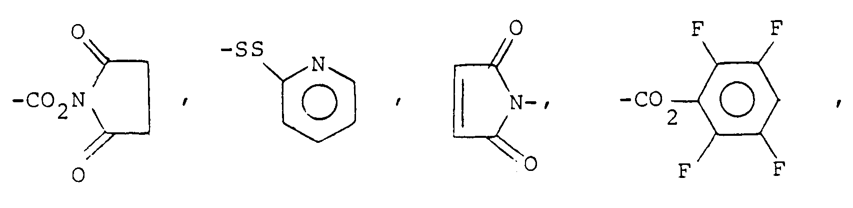 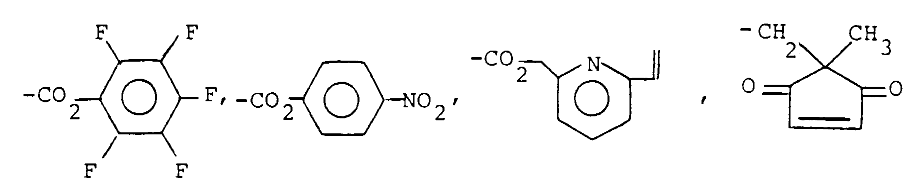 and W is as shown in Table 1, above.

As long as the product from Scheme I contains at least one functional group which can be converted to, or is directly reactive with a targeting unit (Tu), targeted forms of the antitumor antibiotics of the above-named patents and applications can be generated, as shown in Scheme II below: 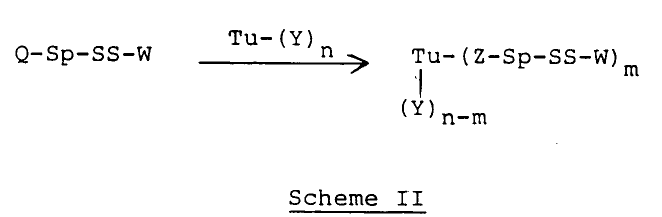 wherein Q, Sp, and W are as hereinbefore defined, Tu is a mono- or polyclonal antibody, its fragments, its chemically or genetically manipulated counterparts, growth factors, or steroids; Y is a side-chain amino, carboxy, or thiol group of a protein, an aldehyde derived from carbohydrate residues, or an amidoalkylthio group; n is an integer of from 1 to 100; Z is formed from covalent reaction of the groups Q and Y directly or after subsequent reduction and Z is -CONH-, -CONHN=CH-, -CONHNHCH₂-, -NHCONHN=CH-, -NHCONHNHCH₂-,-NHCSNHN=CH-, -NHCSNHNHCH₂-, -ON=CH-, -NH-, -NHCH₂-, -N=CH-, -C0₂-, -NHCH₂C0₂-, -SS-, 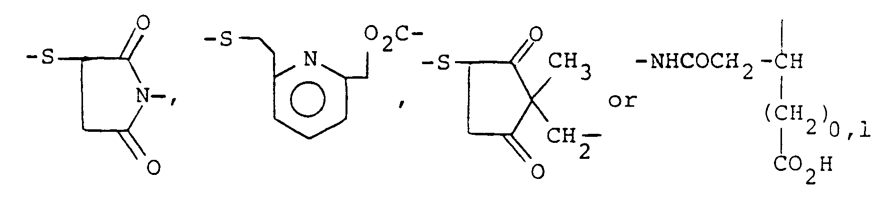 and m is 0.1 to 15.

As an example, and with reference to Scheme II, above, the 3-mercaptopropionic acid derivative of E-33288γ₁^{l} (Q = C0₂ H, Sp =-CH₂CH₂-), when converted to its activated hydroxysuccinimide form (Q = C0₂ Su, Sp=-CH₂-CH₂-) can be used to react with some of the _{E}-amino groups of lysine residues (e.g., Tu=monoclonal antibody, Y = -NH₂ wherein n=50-100 from available lysine residues), at a pH between 7.0 and 9.5 in aqueous buffered solutions at temperatures between 4 °C to 40 °C to produce targeted forms of the antibiotics attached at random sites along the protein backbone (Tu = monoclonal antibody, Z = -NHCO-, Sp=-CH₂CH₂-, m=1-10). Only a fraction of the available lysine residues are substituted in this manner, since high loading is generally not considered compatible with preserving the antibody immunoreactivity. The same randomly-substituted immunoconjugates can also be prepared from the 3-mercaptopropionic acid derivative using other carboxyl group activating agents such as a variety of carbodiimides, or the corresponding acyl azide. Alternatively, a 3-mercaptopropionyl hydrazide derivative of E-33288γ₁^{l} (Q=H₂NNHCO-, Sp=-CH₂CH₂-), when reacted with a periodate-oxidized antibody (Tu=monoclonal antibody, Y=-CHO, n = 1-15) as described in U.S. Patent No. 4,671,958 at a pH between 4 and 7, in a buffered aqueous solution at a temperature of between 4 °C and 40 °C, reacts only at the aldehyde functionality (derived from cleavage of vic-diols of carbohydrate residues situated on the Fc portion of the antibodies) to generate monoclonal antibody conjugates containing the drug substituted at specific sites along the backbone of the protein (Tu=monoclonal antibody, Z=-CH=NNHCO-, Sp=-CH₂CH₂-, m=0.5-10). In order to block unreacted aldehyde groups on the antibody and thus avoid crosslinking, as well as stabilize the hydrolytically labile Schiff's base linkages, it is preferable (though not essential) to react the latter conjugate first with a compound such as acetyl hydrazide or tyrosine hydrazide, then reduce with sodium cyanoborohydride or sodium borohydride to produce the stabilized constructs of this invention (Tu=monoclonal antibody, Z=-CH₂NHNHCO-, Sp=-CH₂CH₂-, m=0.5-10). Other aldehyde-reactive groups as part of the drug construct are within our invention to generate the products of Scheme II. Such functional groups are preferably, though not limited to, those which react with aldehydes under acidic aqueous conditions. The reactivity of protein lysines under basic conditions is sufficiently great such that their amines compete with the products of Scheme II for available aldehydes of the monoclonal antibody. Alternative aldehyde-reactive groups are, for example, the semicarbazide, the thiosemicarbazide, and the O-substituted hydroxylamine functionalities.

Assembly of targeted forms of the compounds listed in Table 1 is not restricted to the sequence outlined in Scheme II. The targeting unit (Tu) can be first modified to contain a thiol group, which is then reacted with the compounds of Table 1, in accordance with Scheme III below: 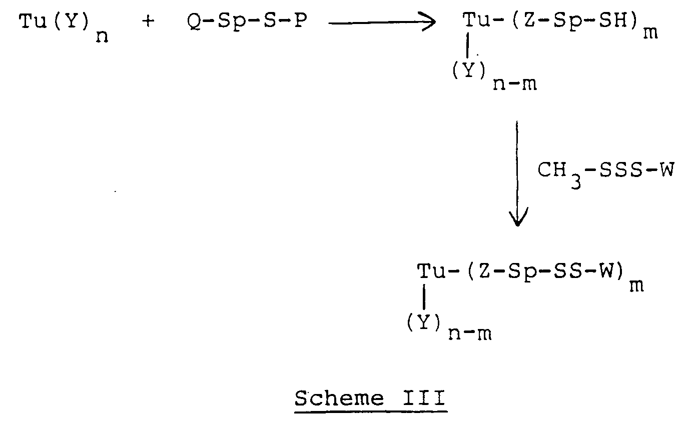 wherein Tu, Y, Q, Sp, W, n, and m are as hereinbefore defined, and P is hydrogen or 2-(pyridylthio), with the proviso that when Y is a thiol derived from a backbone amino acid residue of Tu, Z-Sp taken together is a covalent bond.

As an example, and with references to Scheme III, above, a monoclonal antibody can be reacted with 3-(2-dithiopyridyl)propionic acid hydroxysuccinimide ester to modify the protein through lysine residues (Tu=monoclonal antibody, Y=NH₂, n=50-100, Q=-CO₂Su, Sp=-CH₂-CH₂-, P=2-pyridylthio). Following reduction with, for example, dithiothreitol, an intermediate is generated (Tu = monoclonal antibody, Z =-NHCO-, Sp=-CH₂CH₂-, m=1 to 15) which can be reacted with the compounds of Table 1 to generate the subject immunoconjugates. Similarly, 2-iminothiolane can be reacted with a monoclonal antibody to introduce thiol groups onto the surface of the protein directly, without requiring a reduction step (Tu = monoclonal antibody, Z=-NHCO-, Sp=-(CH₂)₃-, m = to 15), and this intermediate can be reacted with the compounds of Table 1 as before. Alternatively, sulfhydryl groups inherent within the structure of monoclonal antibodies in dimeric form as cystine residues can be used to participate in the reaction of Scheme III directly. Such sulfhydryls are traditionally exposed by a combination of enzymatic digestion and reduction of native monoclonal antibodies (Tu=Fab' fragment, Z-Sp =bond, Y=SH), but the use of genetically-altered constructs of monoclonal antibodies containing unpaired cystine residues is likewise contemplated.

A preferred embodiment of this invention is a protein-drug conjugate of the formula: 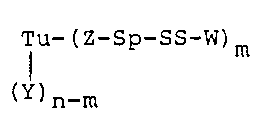 prepared from the antitumor antibiotic designated LL-E33288γ1^{l} (CH3SSS-W) having
a) ultraviolet spectrum as shown in Figure I;
b) a proton magnetic resonance spectrum as shown in Figure II;
c) an infrared spectrum as shown in Figure III; and
   displacing the dithiomethyl moiety with a compound of formula Q-Sp-SH, wherein Sp is straight or branched-chain divalent (C₂-C₅) radicals or divalent (C₂-C₅) arylalkyl or heteroarylalkyl radicals, and Q is carboxyl, lower alkyldicarboxyl anhydride, -C0₂S_{U}, -CONHNH₂, or 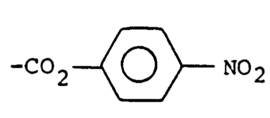 to produce an intermediate of general formula Q-Sp-SS-W, wherein Q, Sp, and W are as hereinbefore defined,
   reacting Q-Sp-SS-W with a molecule of the formula Tu-(Y)ₙ wherein Tu is a monoclonal antibody which exhibits preferential reactivity with a human tumor-associated antigen, Y is a side-chain amino group on the antibody, or an aldehyde generated by oxidation of the carbohydrate groups of the antibody, and n is an integer of from 1 to 100, to produce a compound of the formula: 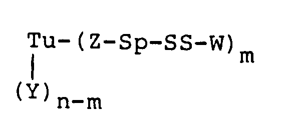 wherein Tu, Y, Sp, W, and n are as hereinbefore defined, and Z is formed from covalent reaction of the groups Q and Y directly or after subsequent reduction, and Z is -CONH-, CONHN = CH-, -CONHNHCH₂-, or 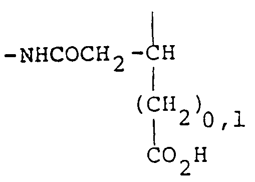 and m is 1 to 15.

A number of different monoclonal antibodies (MoAb's) are used to exemplify targeting of the methyltrithio anticancer compounds. MoAb's Lym 1 and Lym 2 recognize different antigens on mature B-lymphocytes and their product lymphomas. The production and characteristics of these MoAb's are described by A.L. Epstein, et. al., "Cancer Research" 47, 830 (1987). MoAb B72.3 targets primarily to carcinomas of the breast and colon, through reactivity with pancreatic, ovarian, and lung carcinomas has also been noted. The antibody has been described by T.L. Klug, et. al., "Int. J. Cancer" 38, 661 (1986). MoAb CT-M-01, which recognizes primarily breast tumors is described in EPO application 86 401482.4 filed July 3, 1986 and MAC-68 is produced by a sub-clone of the hybridoma which produces CT-M-01, and recognizes both breast and colon carcinomas. Intermediates of the subject compounds useful for, and conjugates with these antibodies, are described in the experimental section. It should not, however, be construed that this patent is limited to or restricted by the aforementioned antibodies. Instead, the methodology is sufficiently general that it can be applied to all antibodies regardless of their class or isotype, their enzymatically-derived fragments, their chemically manipulated and stabilized fragments, as well as their respective chimeric and humanized counterparts. Nor are the targeting units restricted only to monoclonal antibodies. Other proteins, as well as small molecules for which receptors exist on target tissues, are within the purview of our discovery as targeting entities.

The methods of this invention used to produce monoclonal antibody conjugates from the compounds of Table 1 yield constructs which retain good immunoreactivity with target cell lines, as determined by the following in vitro assays:

### Target Cells

All target cells were maintained in RPMI 1640 media supplemented with 5% Fetal Calf Serum (FCS), ITS (Collaborative Research, Cat# 40351), streptomycin (50 µg/ml), penicillin (50 units/ml), gentamycin sulfate (50 µg/ml) and glutamine (.03%). The cells were maintained in a humidified 5% C0₂ incubator at 37 °C.

### I. Immunoreactivity Assays

### Procedure I - Elisa

Appropriate target cells were harvested, counted and suspended in Dulbecco's Phosphate Buffered Saline (DPBS) at an optimal concentration for monoclonal antibody (MoAb) being tested. 0.1 ml of cells was aliquoted in each well of a sterile tissue culture polystyrene 96-well plate. The plates were centrifuged for 5 minutes at 1,000 RPM's and the supernatant was flicked off. Plates were air-dried overnight and may be stored at 4 _{°} C for up to 3 months.

Non-specific binding sites were blocked by adding 200 µl of 1% gelatin in DPBS per well and incubating the plate for 1 hour at 37 _{°} C in a humid incubator. (All subsequent incubations are done under similar conditions). The plates were washed once with 250 µl of 0.05% TWEEN-20 in DPBS (washing solution) using the automated ELISA washing system from Dynatech (Ultrawash II). Samples to be tested were diluted to make a final concentration of 3 µg/ml MoAb equivalents in 0.1% gelatin-DPBS. Six additional threefold serial dilutions were prepared from each 3 µg/ml sample and 100 µl was added to appropriate wells in triplicate. The bottom row of wells only received 100 µl of 0.1% gelatin as background. Plates were incubated for 45 minutes and then washed three times. Alkaline phosphatase conjugated affinity purified goat anti-mouse immunoglobulins (Cappel Cat# 8611-0231) was diluted 1:125 in 0.1% gelatin and 100 µl was added to each well. Plates were incubated for 45 minutes and then washed three times. 200 µl of p-nitrophenyl phosphate substrate solution (see below) was added to each well. After 45 minutes at room temperature the reaction was stopped by the addition of 50 µl of 3M NaOH. The absorbance of the contents of each well was read at 405 nm in the automated spectrophotometer from Dynatech (EIA Autoreader # EL-310).

### Substrate Diethanolamine Buffer (10%)

97 ml diethanolamine
800 ml water
0.2 grams NaN3
100 mg MgCl2 6H2 0

The reagents were dissolved by continuous stirring and 1 M HCI was added until the pH was 9.8. The total volume was made up to 1 liter with water and filter sterilized with a 0.2 µ filter. The buffer was stored in the dark at 4 _{°} C. Immediately before use, p-nitrophenyl phosphate (Sigma, Cat# 104-40) was dissolved in the 10% diethanolamine buffer (must be at room temperature) to give a final concentration of 1 mg/ml.

### Calculation of O. D. Values

The percentage binding of each sample was calculated by the following equation: 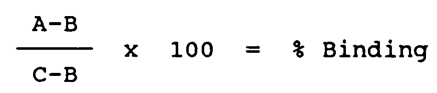
A = Average O.D. of test sample
B = Average O.D. of background
C = Average O.D. of 3 µg/ml unmanipulated MoAb control

The % binding was plotted on the non-log scale of a semi-log graph and the MoAb concentration was plotted on the log scale. The BD₅₀ (i.e. dose of antibody needed to give 50% binding) of each test sample was derived from the graph and the amount of retention of immunoreactivity was calculated by the following equation:

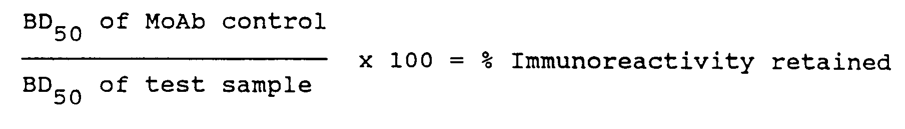

### Procedure 2 - Indirect RIA

Appropriate amounts of target cells in 0.2 ml of 10% FCS media were aliquoted into 4 ml polystyrene tubes. Samples to be tested were diluted to a concentration of 2 µg/ml MoAb equivalents in 10% FCS media. Five three-fold serial dilutions were prepared from each 2 µg/ml sample and 0.2 ml was added to each tube in duplicate. Background samples received only cells and media. Cells were incubated at 4 °C for 1 hour, then washed 2 times (all RIA washes were done with a 3 ml volume) with 2% FCS media. 0.05 ml of sheep F(ab')₂ anti-mouse IgG [¹²⁵1] (Dupont, Cat# NEX 162-0142) containing approximately 500,000 CPM's was added to each tube; cells were incubated an additional hour at 4 °C, washed once with 2% FCS and twice with PBS. 0.5 ml of PBS was added to each tube, cells were vortexed, transferred to clean tubes and counted for 1 minute in a Packard Gamma 500.

The % binding of each value was determined and graphed like the preceding ELISA equation, except CPM's were substituted for O.D. units and C = Average CPM's of 1 µg/ml unmanipulated MoAb control. The % immunoreactivity retained of each sample was calculated as previously discussed.

### Procedure 3 - Direct RIA

Appropriate amounts of target cells in 1 ml of 10% FCS media were aliquoted into 4 ml polystyrene test tubes, centrifuged and supernatant was discarded. Samples to be tested were diluted to make a concentration of 200 µg/ml MoAb equivalents in 10% FCS media. Five additional five-fold serial dilutions were prepared from each 200 µg/ml sample and 0.05 ml was added to each tube in duplicate. 0.05 ml of 125 MoAb was added to each tube (optimal amount is individually determined for each MoAb and batch). Positive control samples contained cells, media and ¹²⁵I-MoAb. Background samples contained non-specific cells, media and ¹²⁵1-MoAb. Cells were incubated for 1 hour at 4 °C, washed once with 2% FCS media, twice with PBS, transferred and counted as previously mentioned.

The % ²⁵¹I-MoAb binding inhibition of each sample was calculated by the following formula: 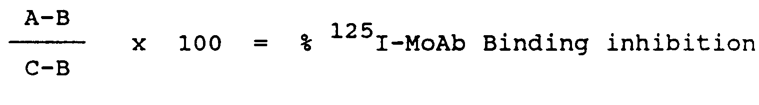
A = Average CPM's of sample
B = Average CPM's of background
C = Average CPM's of positive control

The plot and % immunoreactivity retained by each sample was calculated as previously discussed except the BD₅₀ is actually BID₅₀ (Dose of MoAb needed to give 50% inhibition of the binding of ¹²⁵I-MoAb).

### Notes:

1) Tubes were always vigorously vortexed immediately after the addition of every reagent in the RIA's.
2) An internal control sample equalling 50% of the unmanipulated MoAb control was included in each set of assays to confirm whether each procedure was quantitative in predicting the conjugates' retention of immunoreactivity.

The results from these assays are tabulated below in Table 2.

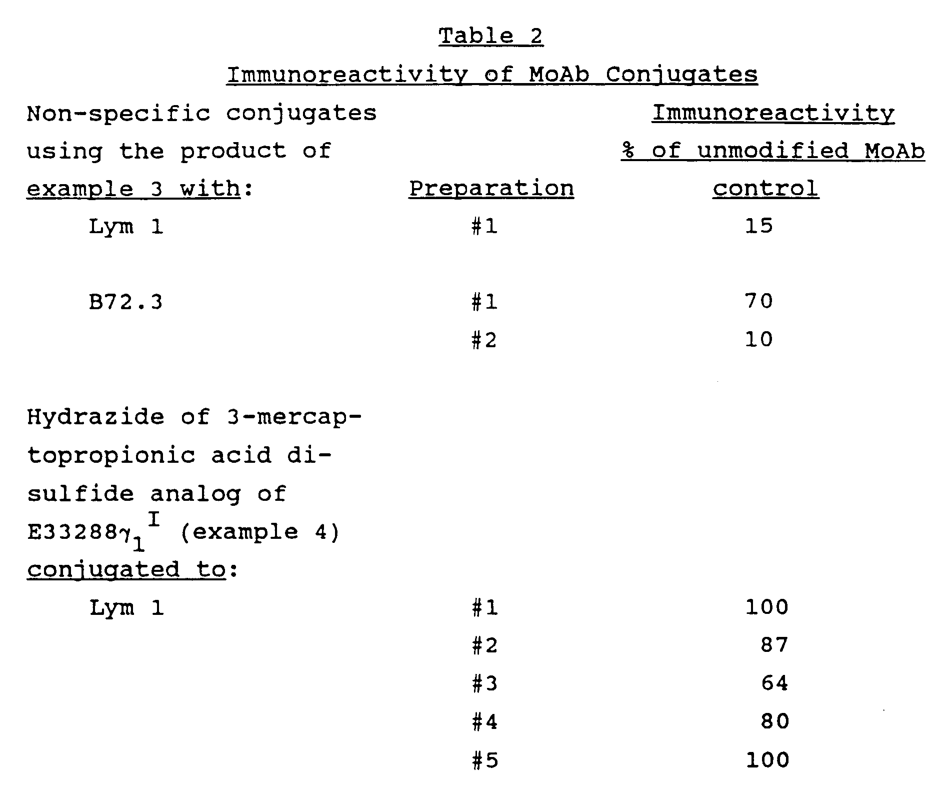

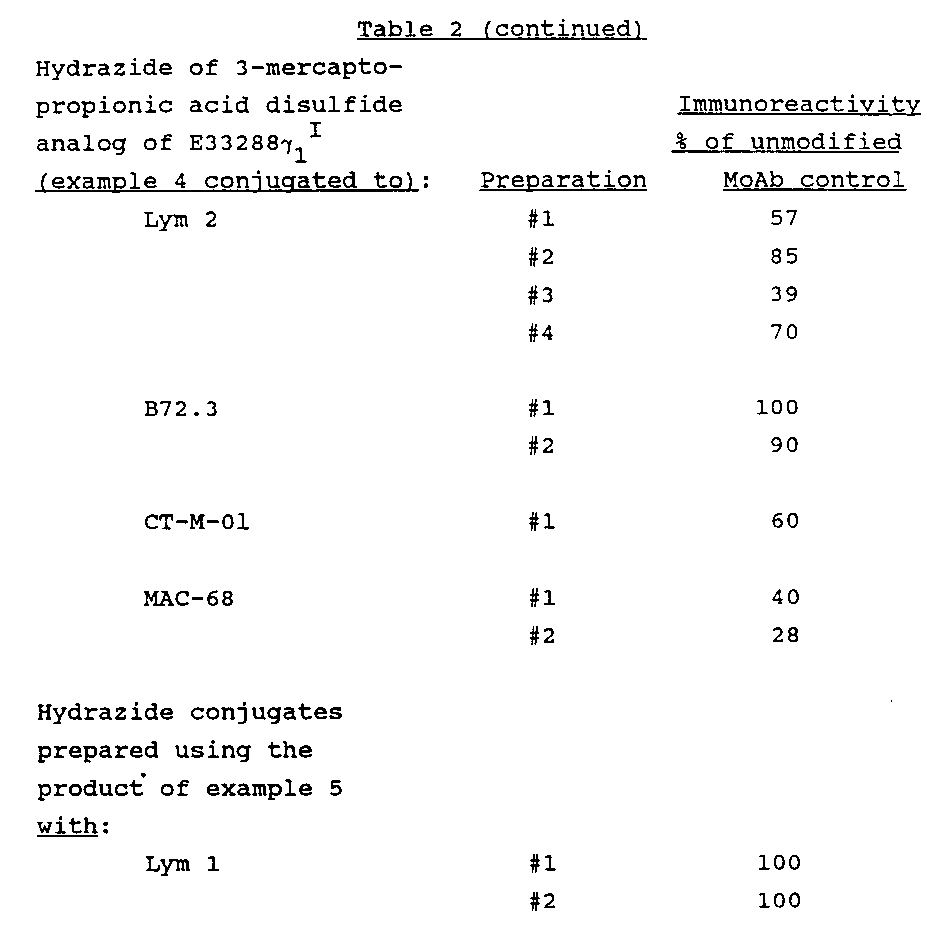

The monoclonal antibody conjugates of this invention are active as anticancer agents. The assay described below for assessing in vitro cytotoxicity shows the dramatic preference of the constructs for target cell lines as opposed to non-target cells, and provides a measure of utility of targeted forms of the compounds compared to their non-targeted counterparts.

### Cytotoxicity Assays

### In Vitro

Samples to be tested were diluted to a concentration of 0.2 or 0.02 µg/ml of LL-E33288γ₁^{l} equivalents (starting concentration is dependent on cell line to be tested). Three additional five-fold dilutions were prepared from each original sample dilution and 0.2 ml was added to sterile 15 ml polystyrene tubes. At least one similar conjugate consisting of LL-E33288γ₁^{l} and an irrelevant MoAb was included in each assay to determine specificity of the relevant conjugate. 10⁵ appropriate target cells in 0.2 ml of 10% FCS media were aliquoted into the tubes and vortexed. In addition, an identical test was performed utilizing irrelevant cells as targets to further confirm specificity of relevant conjugate. MoAb controls received only equivalent amounts of MoAb and positive control samples received only 10% FCS media.

Cells were incubated at 37 _{°} C for 7 minutes then washed 4 times with 8 ml of 2% FCS media. 0.1 ml of 10% FCS was added to each tube, cells were vortexed and 0.2 ml was aliquoted to each well of a sterile 96-well polystyrene tissue culture plate.

Plates were incubated for 2 days in a humidified 37 °C incubator with 5% C0₂. One half of the media was removed and replaced with fresh media containing 2 µCi/ml ³H thymidine (DuPont, NEN, Cat# NET-027). Incubation was continued for 24 hours, cells were frozen, thawed and harvested by a PHD cell harvester (Cambridge Technology, Inc.). Each sample was counted for 1 minute in a Beckman LS 5800 scintillation counter on Channel 1.

The % growth inhibition was calculated as follows: 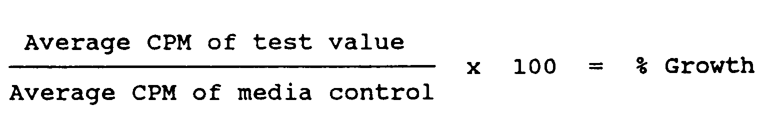 100 - % Growth = % Inhibition

The % inhibition was plotted on the non-log scale of a semi-log graph and the LL-E33288γ,₁^{l} concentration was plotted on the log scale. The IC₅₀ (concentration of LL-E33288γ₁^{l} needed to give 50% inhibition) of each test sample was derived from the graph and the amount of retention of cytotoxicity was calculated by the following equation:

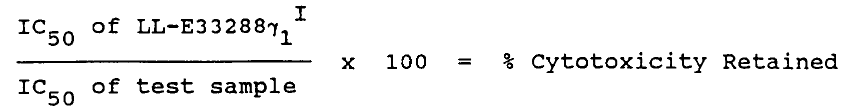

The results from the in vitro cytotoxicity assay are tabulated below in Table 3.

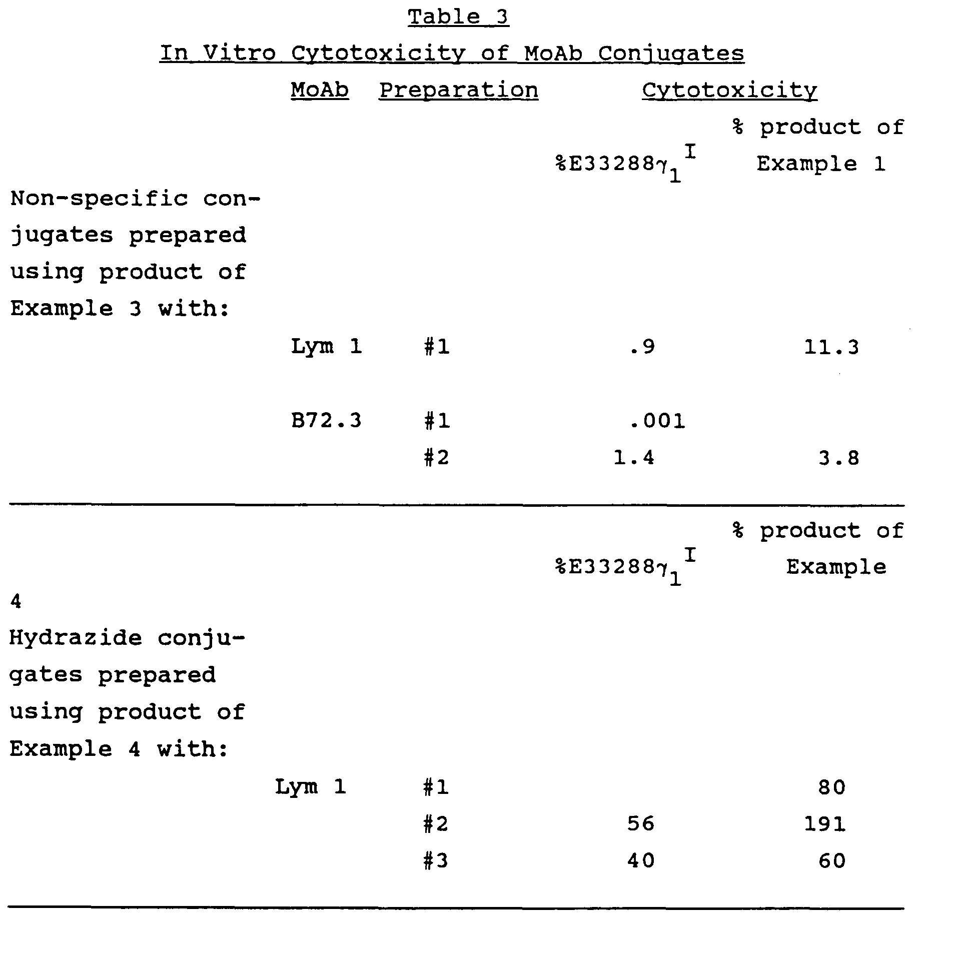

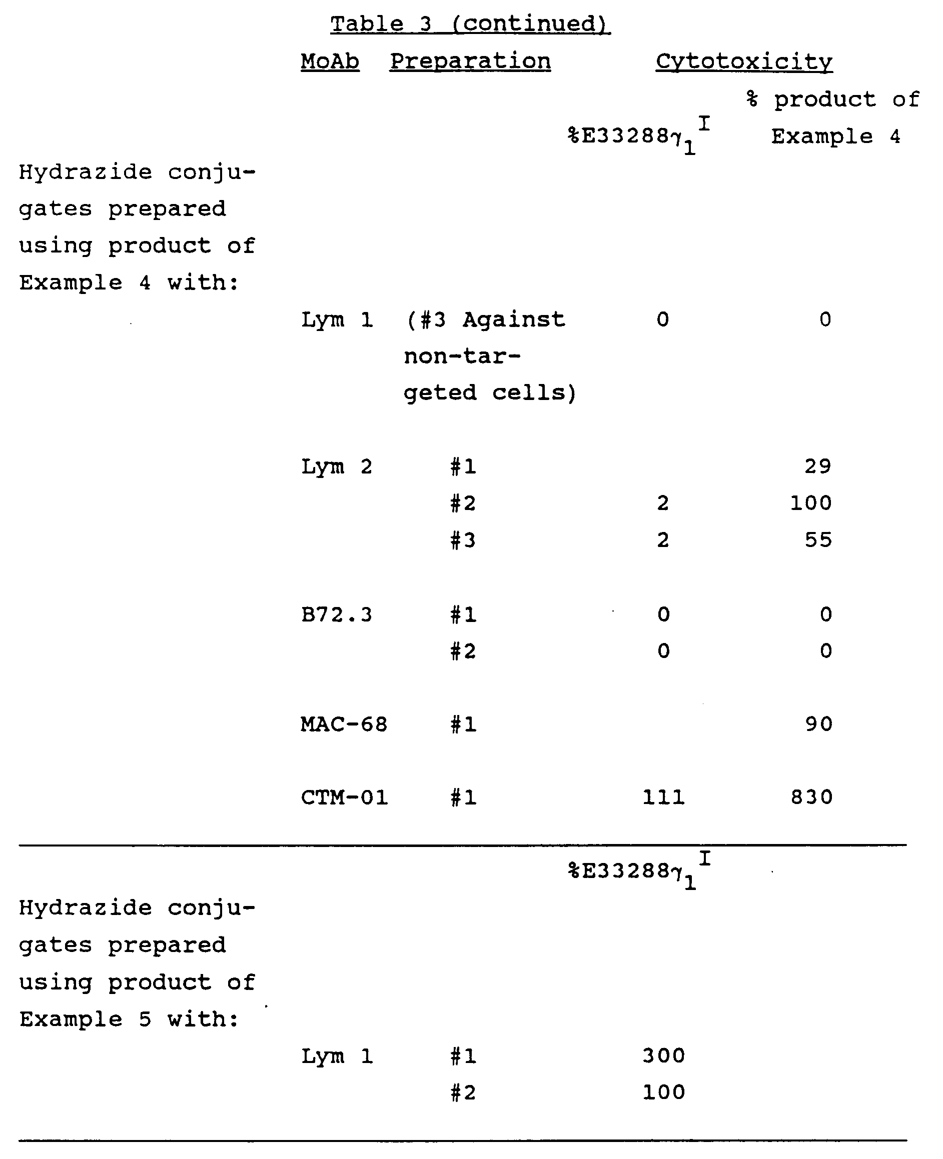

The following assay system was used to measure the in vivo activity of the conjugates of this invention.

In vivo tests for antitumor activity on drug-monoclonal antibody conjugates were done using human tumor xenographs in athymic (nude) mice.

Burkitt lymphoma (Raji) and myeloma (HS Sultan) cells were harvested from culture flasks and inoculated subcutaneously (≧ 80 x 10⁶ Raji cells or 40 x 10⁶ HS Sultan cells) into test mice. Solid tumors, ovarian carcinomas (CA73, Ovcar-3) and breast carcinoma (MX-1) were propagated in athymic mice, removed, cut into 2 mm³ fragments and implanted subcutaneously into test mice (5-8 fragments per mouse).

Drugs, monoclonal antibodies and drug-monoclonal antibody conjugates were administered intraperitoneally once each 3 days for 3 or 5 total injections starting on day 2, 3, 4, 6, 7 or 8 days after tumor implantation. Tumor measurements (the length and width of the tumor) were made by means of a Fowler ultra CAL II electronic caliper each 7 days for 4 or 5 weeks post tumor implantation. Tumor mass in mg was estimated from the formula:

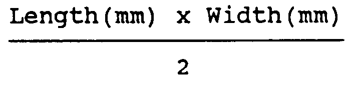

Tumor growth inhibition was calculated for each test group on a percent of control [mean mg of treated (T) divided by mean mg of control (C) x 100]. A T/C value ≦42% in groups with ≧65% surviving animals is considered necessary to demonstrate activity.

The results from this assay appear in Table 4.

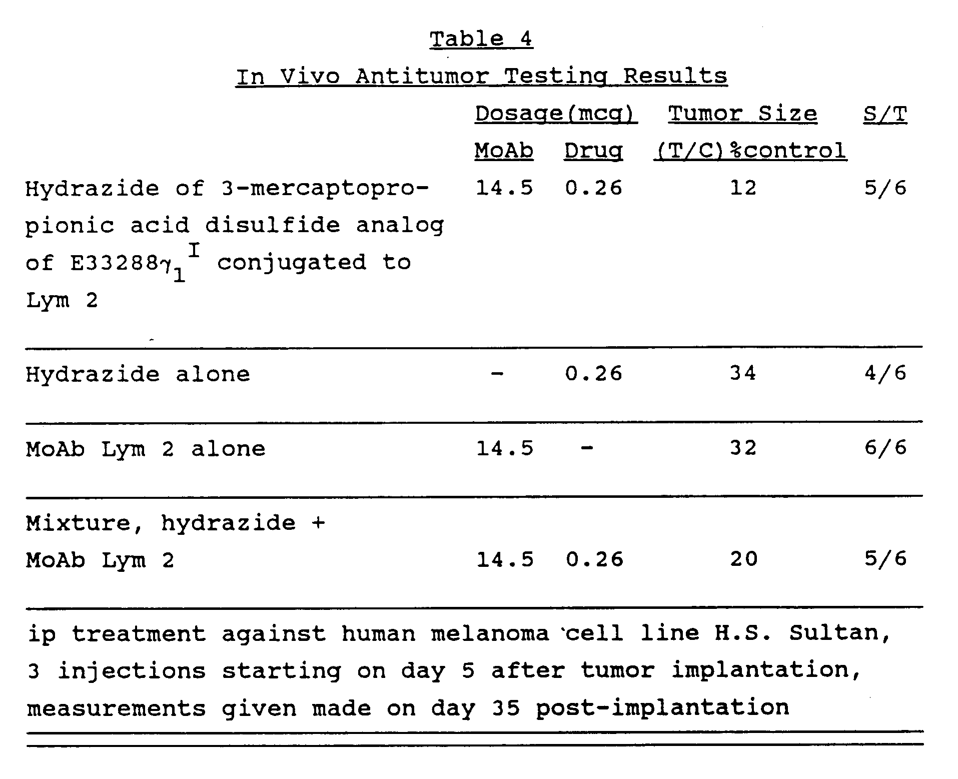

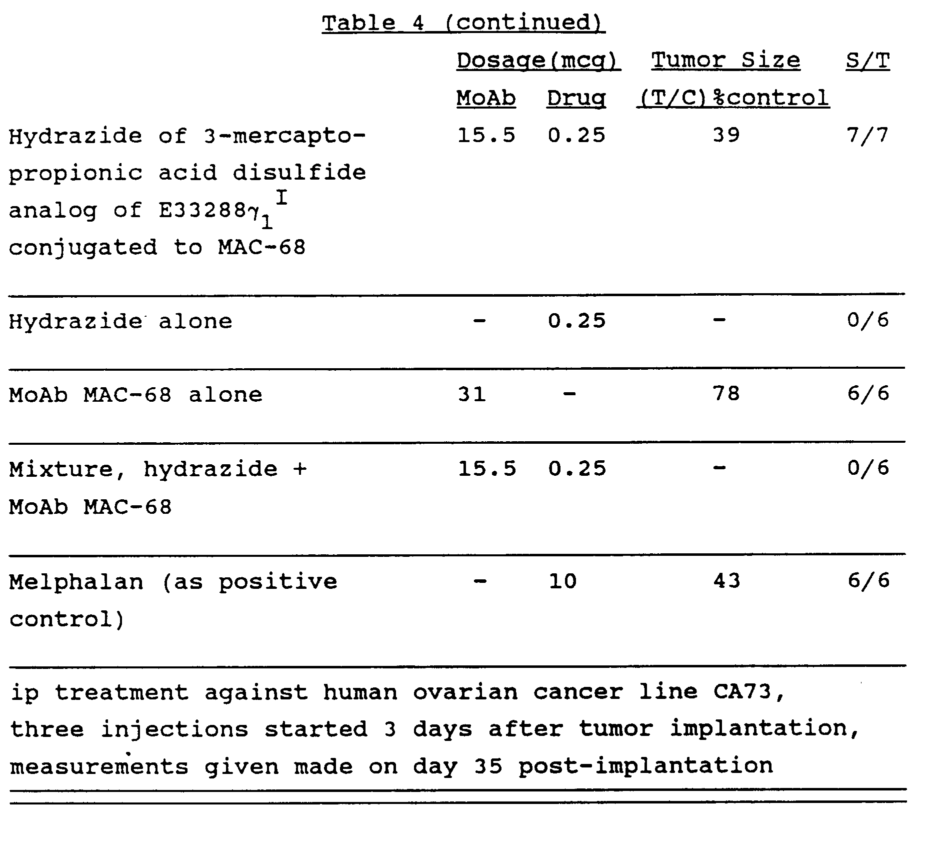

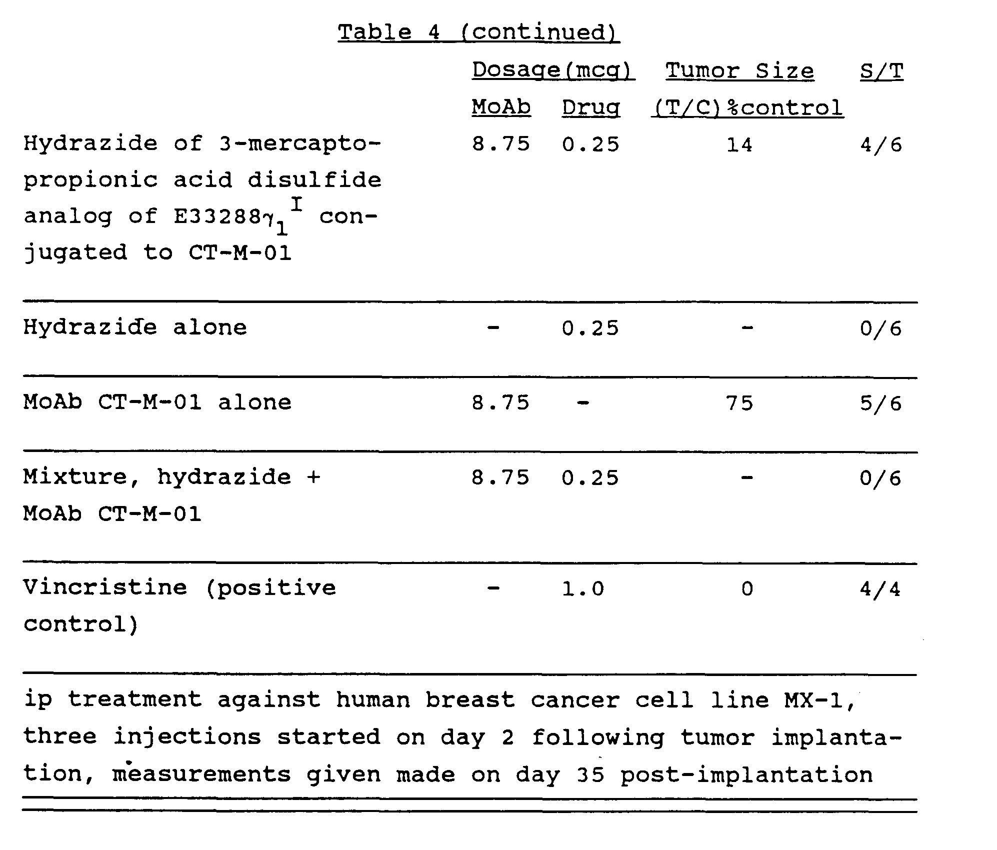

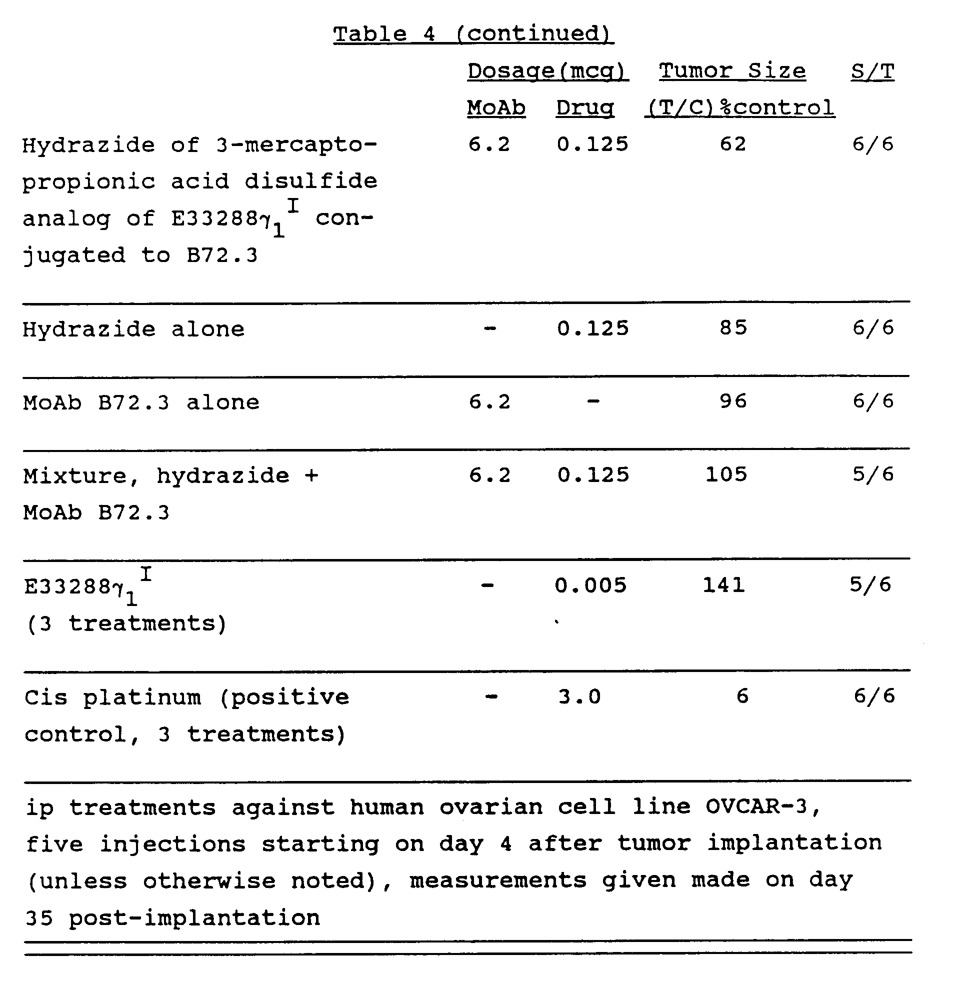

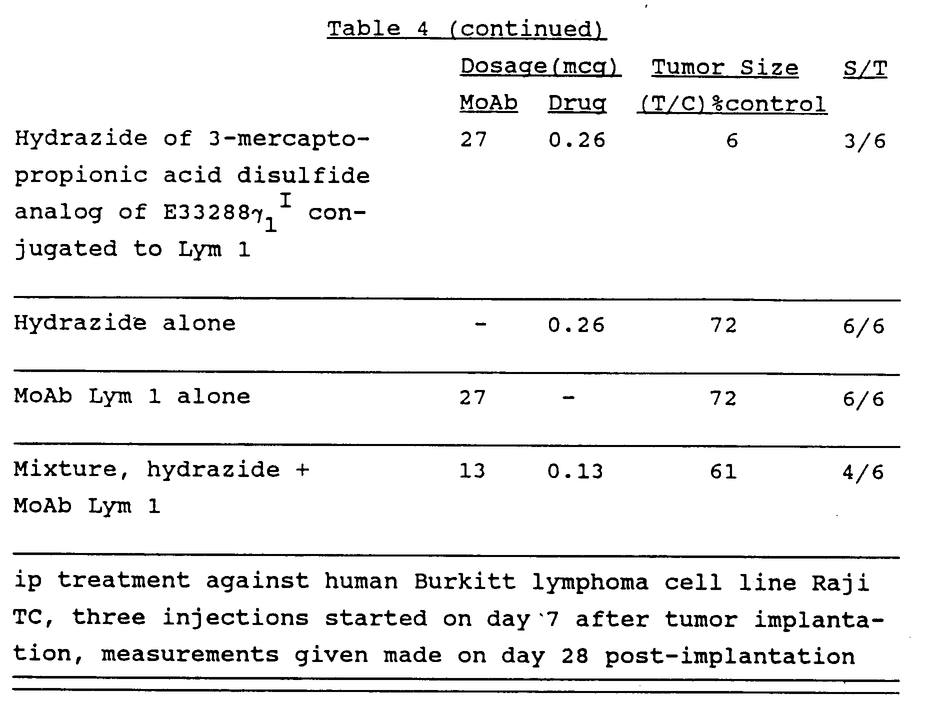

The invention will be further described in conjunction with the following non-limiting examples.

### Example 1

### 3-Mercaptopropionic Acid Disulfide Analog of LL-E33288γ₁^{l}

To a solution of 90 mg of LL-E33288γ₁^{l} in 90 ml of acetonitrile was added 10.6 mg of 3-mercaptopropionic acid in 1 ml of acetonitrile. The solution was vortexed and then stored at -20 _{°} C for 6 days. The solvent was removed in vacuo and the residue chromatographed over 10 ml of silica gel in methylene chloride. The column was developed with 50 ml of methylene chloride, 50 ml of 4% methanol in methylene chloride and finally 100 ml of 8% methanol in methylene chloride. Evaporation of this last fraction gave a residue which was taken up in ethyl acetate with the aid of a little acetone and added dropwise to an excess of hexane. The precipitate was collected and dried, giving 39 mg of the desired product (FABMS, M + H 1394). Retention time on C₁₈ reverse phase HPLC: 18 minutes with 50% acetonitrile/0.1 M aqueous ammonium chloride. (LL-E33288_{γ}1^{l} :8.0 minutes, ester hydrolysis product: 1.5 minutes)

### Example 2

### Reaction of LL-E33288γ₁^{I} with the p-nitrophenyl ester of 3-mercaptopropionic acid

### (A) Preparation of _{R}-nitrophenyl ester of 3-mercaptopropionic acid

Commercial 3-mercaptopropionic acid in methylene chloride containing a catalytic amount of concentrated sulfuric acid was treated with isobutylene for 20 minutes. The solution was then extracted with 1 N sodium bicarbonate solution after which the methylene chloride solution was dried using anhydrous magnesium sulfate. The solution was then evaporated to a colorless mobile liquid which NMR and mass spectral data indicated was the S-t-butylmercaptopropionic acid, t-butyl ester.

An aliquot of this ester was refluxed with 6N hydrochloric acid in dioxane for 2.5 hours. The solvent was evaporated, ethyl acetate was added and this solution was extracted with sodium carbonate. The sodium carbonate extract was treated with 6N hydrochloric acid until the pH of the suspension was 2.0. The suspension was then extracted with ethyl acetate, the extract dried over anhydrous magnesium sulfate and the solvent evaporated to a colorless liquid which 'H NMR and mass spectral data indicated was S-t-butylmercaptopropionic acid.

This compound was converted to the p-nitrophenyl ester by treatment with equimolar amounts of p-nitrophenol and dicyclohexylcarbodiimide in tetrahydrofuran for 4 hours. The dicyclohexyl urea by-product was removed by filtration and the filtrate was evaporated to an oil which was purified by passage over neutral silica gel using the solvent system hexane:methylene chloride (50:50). The pure p-nitrophenyl ester derivative was a faintly yellow, mobile oil.

The free mercaptan was unmasked by the following procedure. The S-t-butylmercaptopropionic acid p-nitrophenyl ester was dissolved in trifluoroacetic acid and a slight molar excess (10%) of mercuric acetate was added. The mixture was stirred for 30 minutes, then the trifluoroacetic acid was evaporated and the residue taken up in dimethylformamide. This solution was treated with hydrogen sulfide gas for 15 minutes, then the black mercuric sulfide was filtered off and the filtrate evaporated under reduced pressure to eliminate up to 99% of the dimethylformamide. The resultant slightly brownish mobile liquid was purified over neutral silica gel using hexane:methylene chloride (50:50). The major component was shown by ¹H NMR to contain a small amount of the t-butyl mercapto derivative. Analytical HPLC over two Perkin-Elmer Pecosphere C₁ a columns in tandem [4.6 x 33 mm and 4.6 x 83 mm] using a gradient system of 37.5/62.5 to 47.5/52.5 of acetonitrile and 0.1 M ammonium acetate buffer at pH 6.5 (acetic acid) over a 12 minute span indicated that the product was 88% of the p-nitrophenyl ester of 3-mercaptopropionic acid and 10% of the less polar S-t-butylmercaptopropionic acid p-nitrophenyl ester. There was also a small amount of free p-nitrophenol present.

### (B) Reaction of p-nitrophenyl ester of 3-mercaptopropionic acid with LL-E33288γ₁^{I}

A 100 mg portion of LL-E33288γ₁^{l} was dissolved in 50 ml of acetonitrile. To this was added a solution of 25.7 mg of p-nitrophenyl ester of 3-mercaptopropionic acid in 1 ml of acetonitrile. The reaction was left at -20 °C for 48 hours. HPLC indicated the reaction was complete. The solution was evaporated to dryness and the residue taken up in 4-5 ml of ethyl acetate using sonication to effect solution. The mixture was filtered and the filtrate dripped into 45 ml of stirred hexane. The resultant faintly yellow solid was collected and dried under reduced pressure, giving 93 mg of the p-nitrophenyl ester of propionic acid derivative of LL-E33288γ₁^{l} as established by ¹H NMR. By FABMS the [M + H] ion appeared at M/Z=1515.

### Example 3

### N-Hydroxysuccinimidyl 3-mercaptopropionate disulfide analog of LL-E33288γ₁^{l}

To a solution of 5 mg of the 3-mercaptopropionic acid disulfide analog of LL-E33288γ₁^{l} from Example 1 in 0.5 ml of tetrahydrofuran was added 0.45 mg of N-hydroxysuccinimide in 0.1 ml of tetrahydrofuran and then 1.8 mg of dicyclohexylcarbodiimide in 0.2 ml of tetrahydrofuran. The reaction was allowed to stir at room temperature for 4 hours and was then quenched with a large excess of hexanes. The solid was isolated by filtration and dissolved in ethyl acetate. The resulting solution was washed three times with brine, dried with magnesium sulfate, and evaporated to 5 mg of the desired product as a tan powder which was used without further purification. Retention time on reverse phase C₁₈ HPLC: 15 minutes with 40% acetonitrile/0.1 M aqueous ammonium chloride (starting material: 6.0 minutes).

### Example 4

### 3-Mercaptopropionyl hydrazide disulfide analog of LL-E33288_{γ1}^{l}

To 5.4 ml (3 eq) of anhydrous hydrazine in 100 ml of refluxing tetrahydrofuran under argon was added dropwise 9.2 ml (83 mmol) of methyl 3-mercaptopropionate in 50 ml tetrahydrofuran over 2 hours. The solution was refluxed an additional two hours, evaporated, and then diluted and evaporated twice from 300 ml of toluene. The product was applied to a plug of silica gel with 5% ethyl acetate/chloroform and eluted from the plug with 20% methanol/chloroform. The resultant 3-mercaptopropionyl hydrazide was a faintly pink oil which solidified when cooled but melted at room temperature.

To 50 mg of LL-E33288γ₁^{l} in 50 ml of acetonitrile at -15°C was added 6.6 mg of 3-mercaptopropionyl hydrazide in 1 ml tetrahydrofuran. One equivalent of triethylamine and/or one equivalent of acetic acid was added as catalyst. The reaction was allowed to stir at 0 _{°} C for one hour and the solvent was then evaporated. The residue was chromatographed on silica gel with a 10-15% methanol in chloroform gradient to yield 26 mg of the desired product. Retention time on reverse phase C₁₈ HPLC: 5.0 minutes in 41% acetonitrile/0.1 M aqueous ammonium chloride.

### Example 5

### N-[[(4-Methyl-coumarin-7-yl)amino]acetyl]cysteine hydrazide disulfide analog of LL-E33288γ₁l

A mixture of 1.0 g (5.7 mmol) of 4-methyl-7-amino-coumarin, 3.0 ml of ethyl bromoacetate (5 eq), 90 mg (0.1 eq) of sodium iodide, and 30 ml dimethylformamide was heated under argon at 80 _{°} C for 5 hours. The mixture was cooled, diluted with ethyl ether, washed three times with 50% brine, dried with magnesium sulfate, and evaporated to dryness. The crude product was dissolved in chloroform containing 1% ethyl acetate and filtered through a plug of silica gel. Recrystallization from diethyl ether containing a trace of chloroform yielded pure ethyl N-[(4-methyl-coumarin-7-yl)amino]acetate.

To 1.96 g (7.5 mmol) of the above ester in 15 ml of methanol and 15 ml of tetrahydrofuran was added 10 ml of 1 N aqueous sodium hydroxide. After 30 minutes, 4 ml of 10% aqueous hydrochloric acid was added. The organic solvents were evaporated and the resultant crystalline product was filtered and washed with cold ethanol and then ether. This material was dissolved in 20 ml of tetrahydrofuran and 4 ml of dimethylformamide. Dicyclohexylcarbonyldiimidazole (1.3 g, 2.2 eq) was added and the reaction allowed to stir for 15 minutes. Cysteine ethyl ester hydrochloride (1.6 g, 2.5 eq) and triethylamine (1.2 ml) were then added. After a further three hours, the reaction was diluted with ethyl ether containing 5% methylene chloride and washed once with 10% aqueous hydrochloric acid and twice with brine. After drying with magnesium sulfate and evaporating the solvents, the crude product was crystallized by dissolving in chloroform containing a minimal amount of ethanol and then adding an excess of ether. The crystals were filtered and dried to give pure N-[[(4-methyl-coumarin-7-yl)amino]acetyl]cysteine ethyl ester.

A mixture of 5 ml of chloroform, 20 ml of methanol, and 0.4 ml of hydrazine hydrate were heated to reflux under argon. To this was added 550 mg of N-[[(4-methyl-coumarin-7-yl)amino]acetyl]cysteine ethyl ester. After refluxing for 9 hours the mixture was cooled and the solid product was filtered and washed with chloroform and then ethyl ether. The crude product (which contained thiol and disulfide) was dissolved in dimethylformamide containing dithiothreitol and triethyl amine. After 30 minutes the product was precipitated with excess ethyl ether and collected by filtration. This material was purified further by recrystallization from degassed acetonitrile containing dithiothreitol and a trace of triethyl amine to give pure N-[[(4-methyl- coumarin-7-yl)amino]acetyl]cysteine hydrazide.

To 12 mg of 70% pure LL-E33288γ₁^{l} in 12 ml acetonitrile at 0 _{°} C was added 4 mg of N-[[(4-methyl- coumarin-7-yl)amino]acetyl]cysteine hydrazide in 1.2 ml dimethylformamide. After stirring overnight another 2 mg of N-[[(4-methyl-coumarin-7-yl)amino]acetyl]cysteine hydrazide in 0.6 ml dimethylformamide was added. The reaction was stirred for 3 days at 0 °C and filtered. The acetonitrile was evaporated and the resultant dimethylformamide solution was diluted with an excess of 1:1 hexanes/ether. The product was isolated by filtration and further purified by chromatography on silica gel with a 15-20% gradient of methanol in chloroform to yield 3 mg of the desired product. Retention time on reverse phase C₁₈ HPLC: 3.5 minutes using 45% acetonitrile/0.1 M aqueous ammonium chloride.

### Example 6

### 3-Mercaptopropionyl hydrazide disulfide analog of LL-E33288α₃

To 10 mg of LL-E33288α₃^{l} in 9 ml of acetonitrile at -15°C was added 6.6 mg of 3-mercaptopropionyl hydrazide in 1 ml acetonitrile. One equivalent of triethylamine and/or one equivalent of acetic acid were added as a catalyst. The reaction was allowed to stir at 0 _{°} C for one hour and the solvent was then evaporated. The residue was chromatographed on silica gel with a 10-15% methanol in chloroform gradient to give the desired product. Retention time on reverse phase C₁₈ HPLC: 3.5 minutes in the system 45% acetonitrile/0.1 M aqueous ammonium chloride.

### Example 7

### Non-specific conjugation to proteins

The hydroxysuccinimide ester described in Example 3 was covalently attached to antibodies under slightly alkaline conditions. The following is a general procedure used to make the antibody conjugates listed in Table 5. Antibody at a concentration of 3-5 mg/ml in phosphate buffer containing 0.1 M sodium chloride, pH 7.5 was reacted with a 5-20-fold molar excess of the product from Example 3 with stirring, at room temperature for from 1-4 hours. The conjugated protein was desalted chromatographically and aggregated protein was separated from monomeric material by gel filtration HPLC. Monomeric fractions were pooled and concentrated.

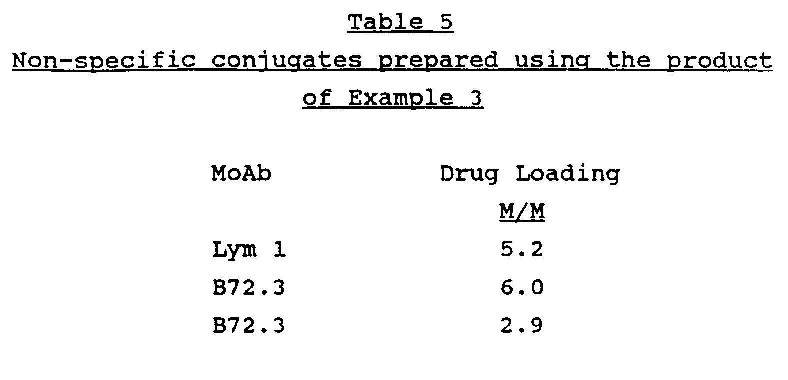

### Example 8

### Site-specific conjugate preparation

The general method for attaching hydrazide derivatives of drugs to oxidized antibodies is described in T. J. McKearn, et al., in U.S. Patent No. 4,671,958. The procedure has been applied to preparing antibody conjugates from the products of Examples 4 and 5 with specific modifications as described below. The products from these reactions and their characteristics are summarized in Table 6.
(A) Antibody Oxidation Antibody at a concentration of 5 to 10 mg/ml was dialyzed overnight against a 200 fold volume of 50mM sodium acetate buffer, pH 5.5 containing 0.1 M sodium chloride (Buffer A). After dialysis, the MoAb was oxidized with 15mM to 200mM periodic acid in 0.2M sodium acetate. The oxidation was allowed to proceed in the dark, with stirring, at 4 °C for 45 minutes after which time the oxidized MoAb was desalted on a >5 bed volume Sephadex G-25 column. The degree of oxidation of the antibody was assessed by reaction with p-nitrophenylhydrazine and comparing absorbance of the protein at 280mm vs. p-nitrophenylhydrazine at 395mm.
(B) Drug Hydrazide Conjugation The oxidized MoAb was reacted with 25 to 200-fold molar excess of drug hydrazide. The hydrazides were dissolved into dimethylformamide and added to the aqueous solution of MoAb. To avoid precipitation of MoAb, the final volume of dimethylformamide added did not exceed 10% of the total reaction volume. Reaction was allowed to proceed for 3 hours at room temperature, with stirring. To prevent crosslinking of unreacted aldehydes and subsequent aggregation, a blocking agent, acetyl hydrazide was added in 100-fold molar excess three hours after addition of the drug hydrazide. To stabilize the Schiff's base linkage between aldehyde and drug hydrazide (a hydrazone), the product generally was reduced to an alkyl hydrazine by the addition of 10mM sodium cyanoborohydride, allowing the reaction to proceed for one more hour (total conjugation time - 4 hours). The conjugate was chromatographically desalted and exhaustively dialyzed (minimum time 48 hours) into pH 6.5 phosphate buffer for storage and testing.

Conjugates were analyzed for the presence of aggregates by gel filtration HPLC and for free drug by reverse phase HPLC. Drug loading was determined spectroscopically using the extinction coefficients of both the antibody and the drug to estimate molar concentrations of drug in conjugates.

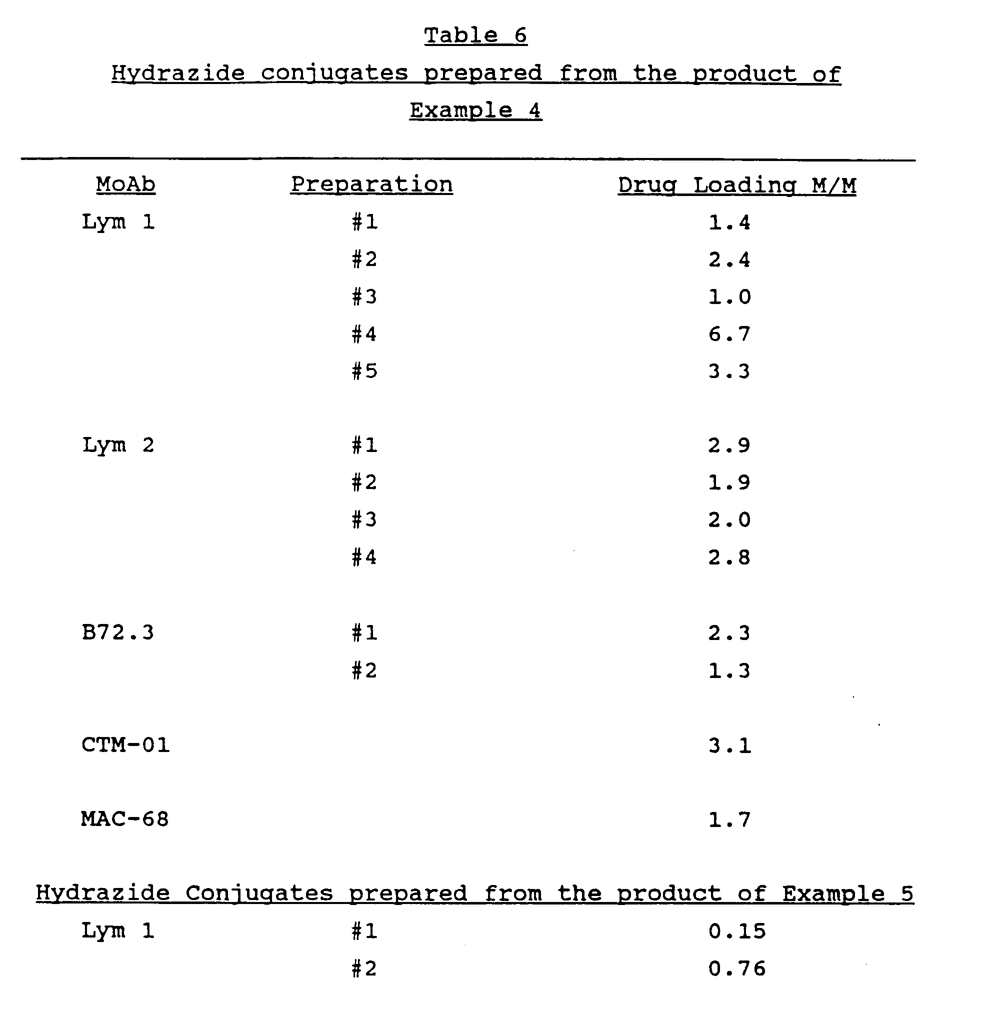

## Claims (Claims for the following Contracting State(s) : AT, BE, CH, DE, FR, GB, IT, LI, NL, SE)

1. A carrier-drug conjugate of the formula 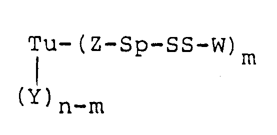 prepared from a compound of formula CH₃SSS-W wherein CH₃SSS-W is an antitumor antibiotic designated as LL-E33288α₁^{Br}, α₁^{l}, α₂Br, α₂^{l} α₃Br, α3^{l}, α₄Br β₁^{Br}, β₁^{l}, β₂^{Br}, β₂l, γ₁^{Br,} γ₁^{l}, δ₁^{l}, BBM-1675, FR-900405, FR-900406, PD 114759, PD 115028, CL-1577A, CL-1577B, CL-1577D, CL-1577E, or CL-1724 comprising:
reacting CH₃SSS-W with a compound of general formula Q-Sp-SH, wherein Sp is a straight or branched-chain divalent (C₁-C₁₈) radical, divalent aryl or heteroaryl radical, divalent (C-C, a ) cycloalkyl or heterocycloalkyl radical, divalent aryl- or heteroaryl-alkyl (C, -C, a ) radicals, divalent cycloalkyl- or heterocycloalkyl-alkyl (C₁-C₁₈) radical or divalent (C₂-C₁₈) unsaturated alkyl radical, and Q is, or can be subsequently converted to, halogen, amino, alkylamino, carboxyl, carboxaldehyde, hydroxy, thiol, a-haloacetyloxy, lower alkyldicarboxyl, -CONHNH₂, -NHCONHNH₂, -NHCSNHNH₂, -ONH₂, -CON3, 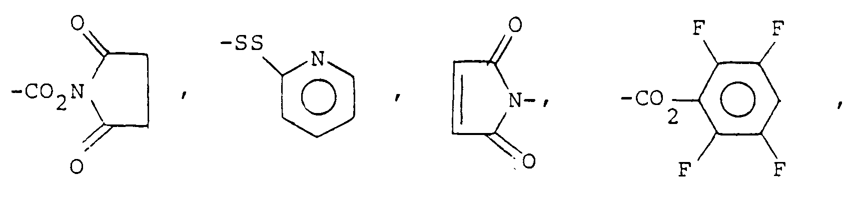 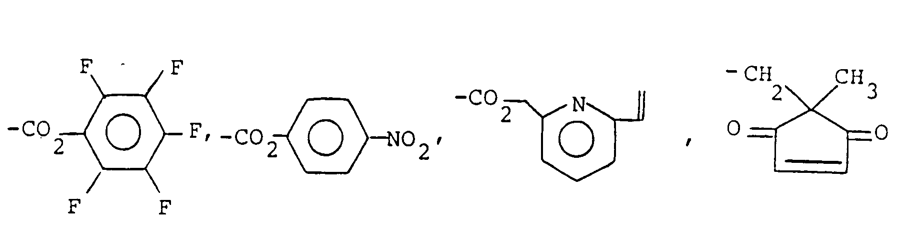 to produce an intermediate of formula Q-Sp-SS-W, wherein Q, Sp, and W are as hereinbefore defined,
reacting Q-Sp-SS-W with a molecule of the formula Tu-(Y)ₙ wherein Tu is defined as a mono- or polyclonal antibody, its fragments, its chemically or genetically manipulated counterparts, growth factors, or steroids; Y is a side-chain amino, carboxy, or thiol group of a protein, an aldehyde derived from carbohydrate residues, or an amidoalkylthio group; and n is an integer of from 1 to 100, to produce a compound of the formula: 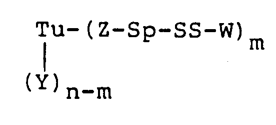 wherein Tu, Y, Sp, W, and n are as hereinbefore defined, and Z is formed from covalent reaction of the groups Q and Y directly or after subsequent reduction, and Z is -CONH-, -CONHN=CH-, -CONHNHCH₂-, -NHCONHN=CH-, -NHCONHNHCH₂-,-NHCSNHN = CH-, -NHCSNHNHCH₂-, -ON=CH- , -NH-, -NHCH₂-, -N=CH-, -C0₂-, -NHCH₂C0₂-, -SS-, 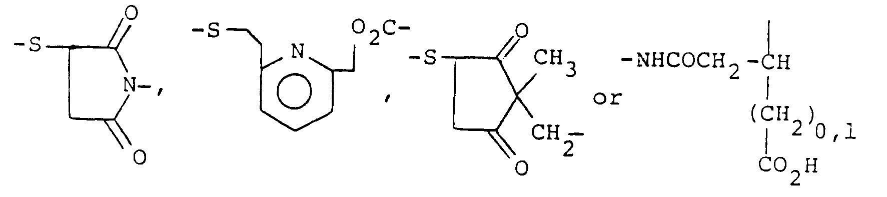 and m is 0.1 to 15.

2. A protein-drug conjugate of the formula 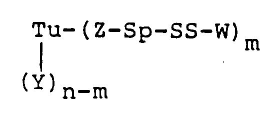 prepared from the antitumor antibiotic designated LL-E33288γ₁^{l} (CH3SSS-W) having
a) ultraviolet spectrum as shown in Figure I;
b) a proton magnetic resonance spectrum as shown in Figure II; and
c) an infrared spectrum as shown in Figure III;
comprising:
displacing the dithiomethyl moiety with a compound of formula Q-Sp-SH, wherein Sp is straight or branched-chain divalent (C₂-C₅) radicals or divalent aryl- or heteroarylalkyl (C₂-C₅) radicals, and Q is, or can be subsequently converted to, carboxyl, lower alkyldicarboxylanhydride, -CONHNH₂, or 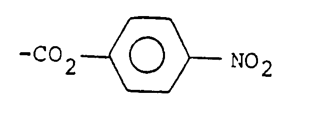 to produce an intermediate of general formula Q-Sp-SS-W, wherein Q, Sp, and W are as hereinbefore defined,
reacting Q-Sp-SS-W with a molecule of the formula Tu-(Y)ₙ wherein Tu is a monoclonal antibody which exhibits preferential reactivity with a human tumor-associated antigen, Y is a side-chain amino group on the antibody, or an aldehyde generated by oxidation of the carbohydrate groups of the antibody, and n is an integer of from 1 to 100, to produce a compound of the formula: 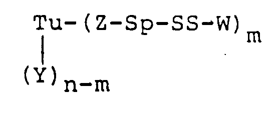 wherein Tu, Y, Sp, W, and n are as hereinbefore defined, and Z is formed from covalent reaction of the groups Q and Y directly or after subsequent reduction, and Z is -CONH-, -CONHN=CH-, -CONHNHCH₂-, or 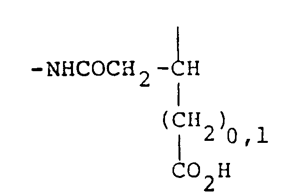 and m is 0.1 to 15.

3. A compound according to Claim 1 wherein CH₃SSSW is LL-E33288γ₁^{l}, Q is the 4-nitrophenyl ester of a carboxyl group, Sp is -CH₂CH₂-, Tu is the monoclonal antibody CT-M-01, Y is -NH₂, Z is -CONH-, and m is 0.5 to 15.

4. A compound according to Claim 1 wherein CH₃SSSW is LL-E33288_{γ1}^{l}, Q is the hydroxysuccinimide ester of a carboxyl group, Sp is -CH₂CH₂-, Tu is the monoclonal antibody MAC-68, Y is -NH₂, Z is -CONH-, and m is 0.5 to 15.

5. A compound according to Claim 1 wherein CH₃SSSW is LL-E33288_{γ1}^{l}, Q is -CONHNH₂, Sp is -CH₂CH₂-, Tu is the monoclonal antibody Lym 1, Y is -CHO, Z is -CONHNHCH₂-, and m is 0.1 to 10.

6. A compound according to Claim 1 wherein CH₃SSSW is LL-E33288_{γ1}^{l}, Sp is 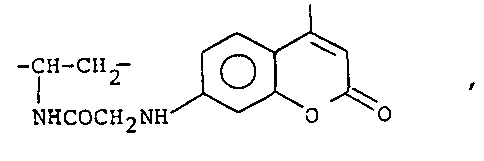 Tu is the monoclonal antibody B72.3, Y is -CHO, Z is -CONHNHCH₂-, and m is 0.1 to 10.

7. A compound according to Claim 1 wherein CH₃SSSW is LL-E33288_{γ1}^{l}, Sp is 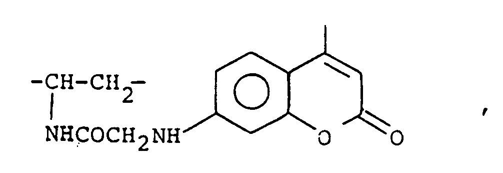 Tu is the monoclonal antibody Lym 2, Y is -CHO, Z is -CONHNHCH₂-, and m is 0.1 to 10.

8. A process for preparing tHe targeted derivatives 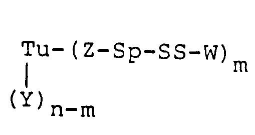 of compounds of formula CH₃SSS-W, wherein CH₃SSS-W is an antitumor antibiotic LL-E33288α₁, ^{Br}, α₁^{l}, α₂^{Br}, α₂^{l}, α₃^{Br} α₃^{l}, α₄^{Br}, β₁^{Br}, β₁^{l}, β₂^{Br}, β₂l, γ₁ Br, γ₁l_{,} δ₁l, BBM-1675, FR-900405, FR-900406, PD 114759, PD 115028, CL-1577A, CL-1577B, CL-1577D, CL-1577E, or CL-1724, comprising
reacting CH₃SSS-W with a compound of formula Q-Sp-SH, wherein Sp is a straight or branched-chain divalent (C₁-C₁₈) radical, divalent aryl or heteroaryl radical, divalent (C₃-C₁₈) cycloalkyl or heterocycloalkyl radical, divalent aryl- or heteroaryl-alkyl (C₁-C₁₈) radicals, divalent cycloalkyl- or heterocycloalkyl-alkyl (C₁-C₁₈) radical or divalent (C₂-Cₗ₈) unsaturated alkyl radical, and Q is halogen, amino, alkylamino, carboxyl, carboxaldehyde, hydroxy, lower alkyldicarboxyl anhydride, -CONHNH₂, -NHCONHNH₂, -NHCSNHNH₂, -ONH₂, or 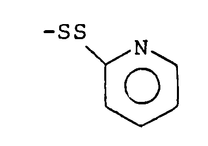 in acetonitrile in the presence of one equivalent of triethylamine and/or one equivalent of acetic acid at -10 to -30 _{°} C for 1-48 hours,
isolating the intermediate of formula Q-Sp-SS-W, wherein Q, Sp, and W are as hereinbefore defined, then
reacting the compound of formula Q-Sp-SS-W, wherein Sp and W are as hereinbefore defined and Q is halogen, amino, alkylamino, carboxyl, carboxaldehyde, hydroxy, or lower alkyldicarboxylic anhydride with a molecule of the formula Tu-(Y)ₙ wherein Tu is a mono- or polyclonal antibody, its fragments, its chemically or genetically manipulated counterparts, growth factors, or steroids; Y is a side-chain amino or carboxy functionality; n is 1-100, in aqueous buffer at a pH of between 6.5 and 9, at 4_{°} to 40 °C either directly or in the presence of a water-soluble carbodiimide, to generate the compound 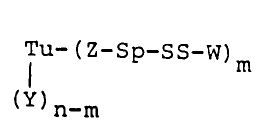 wherein Tu, Sp, W, n, and Y are as hereinbefore defined, m is 1-15 and Z is formed from covalent reaction of the groups Q and Y and is -CONH-, -NH-, 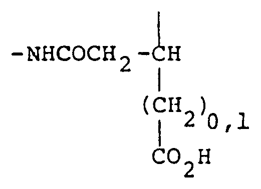 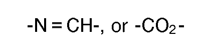 or
reacting the compound of formula Q-Sp-SS-W, wherein Sp and W are as hereinbefore defined and Q is a carboxylic acid, with N-hydroxysuccinimide, 2,3,5,6-tetrafluorophenol, pentafluorophenol, or 4-nitrophenol in the presence of a carboxyl activating agent such as a carbodiimide to generate a compound of formula Q-Sp-SS-W wherein Sp and W are as hereinbefore defined and Q is 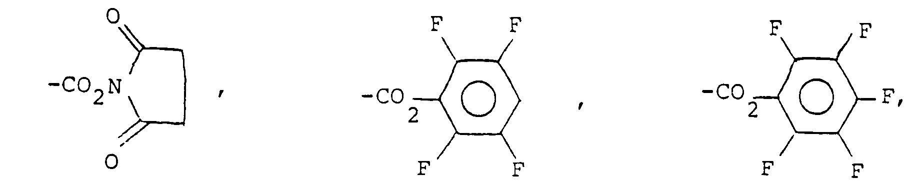 or 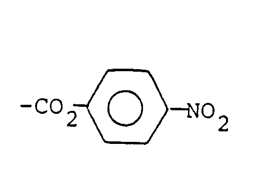 with a molecule of formula Tu-(Y)ₙ,
where Tu and n are as hereinbefore defined, and Y is a side-chain amino group, in an aqueous buffered solution at a pH between 6.5 and 9, at a temperature of between 4 _{°} and 40 _{°} C, inclusive, to generate compounds of the formula: 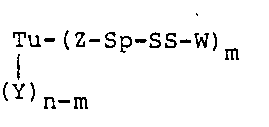 wherein Tu, Sp, Y, and n are as hereinbefore defined, m is 1-15, and Z is formed from covalent reaction between Q and Y and is defined as -CONH-or
reacting a compound of formula Q-Sp-SS-W, wherein Sp and W are as hereinbefore defined and Q is -CONHNH₂ with nitrous acid in aqueous acetonitrile to generate a compound of formula Q-Sp-SS-W, wherein Sp and W are as hereinbefore defined and Q is -CON₃ with a compound of formula Tu-(Y)ₙ, wherein Tu, Y, and n are as hereinabove defined to produce a compound of the formula 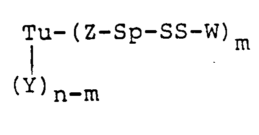 wherein Tu, Z, Sp, W, m, Y, and n are as hereinabove defined; or
reacting a compound of formula Q-Sp-SS-W wherein Sp and W are as hereinbefore defined and Q is hydroxy, with an alpha-haloacetic anhydride to produce a compound wherein Q is a-haloacetyloxy, and reacting the a-haloacetyloxy-Sp-SS-W or a compound of formula Q-Sp-SS-W, wherein Sp and W are as hereinbefore defined and Q is 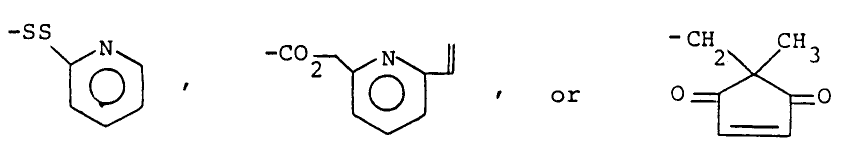 with a molecule of the formula Tu-(Y)ₙ wherein Tu is as hereinbefore defined, Y is a side-chain thiol of a protein, or an amidoalkylthio group introduced on an amine of Tu using reagents such as 3-(2-dithiopyridyl)propionic acid hydroxysuccinimide ester followed by reduction with an agent such as dithiothreitol, or an amidoalkylthio group introduced on an amine of Tu using 2-iminothiolane, and n is 1-10, under aqueous buffered conditions at a pH between 4.5 and 7, at a temperature between 4_{°} and 40 _{°} C, inclusive, to produce a compound of formula: 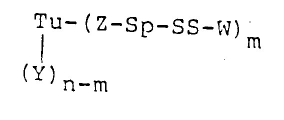 wherein Tu, Sp, W, and n are as hereinbefore defined, and Z is formed from covalent reaction of the groups Q and Y and Z is 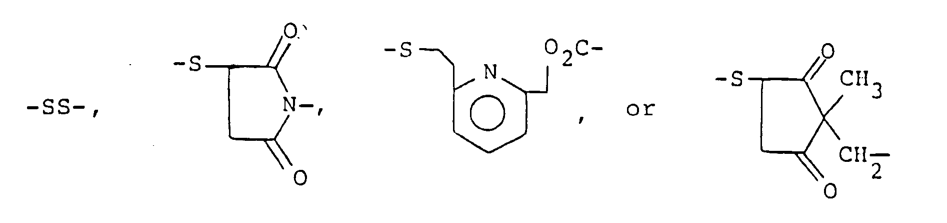 and n is 0.1 to 10; or
reacting a compound of the formula Q-Sp-SS-W wherein Sp and W are as hereinbefore defined and Q is -NH₂, CONHNH₂, -NHCONHNH₂, -NHCSNHNH₂, or -ONH₂ with a molecule of formula Tu-(Y)ₙ wherein Tu is as hereinbefore defined, Y is an aldehyde generated from carbohydrate residues on Tu by oxidation in the presence of an alkaline earth periodate, in an aqueous buffer at a pH between 4.0 and 6.5, at 4 to 40 °C, inclusive, and n is 1 to 20 to generate a compound of formula: 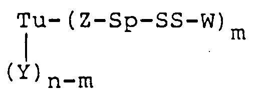 wherein Tu, Sp, W, Y, and n are as hereinbefore defined and Z is formed from the covalent reaction of Q and Y and is -ON = CH-, -N = CH-, -CONHN = CH-, -NHCONHN = CH-, or -NHCSNHN = CH-, and m is 0.1 to 15; or treating the compound immediately hereinabove of formula: 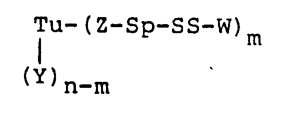 wherein Tu, Z, Sp, W, Y, n, and m are as immediately hereinabove defined with acetylhydrazine or tyrosine hydrazine in an aqueous buffer at a pH between 4.0 and 6.5, at 4° to 40 °C, inclusive, to generate a compound of formula: 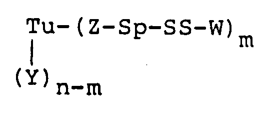 wherein Tu, Z, Sp, W, n, and m are as immediately hereinabove defined and Y is -CH = NNHCOCH₃ or 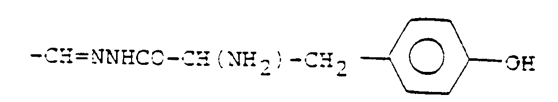 and
reacting this compound with sodium cyanoborohydride or sodium borohydride, in an aqueous buffer at a pH of 4.0 to 6.5, at a temperature of 4° to 40 °C, inclusive, to generate a compound of formula: 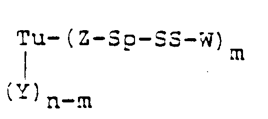 wherein Tu, Sp, W, m, and n are as hereinabove defined, Z is -NH-CH₂-, -CONHNHCH₂-, -NHCONHNHCH₂-, or -NHCSNHNHCH₂-, and Y is -CH₂NHNHCOCH₃ or

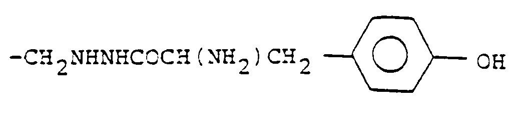

9. A pharmaceutical composition containing a carrier-drug conjugate in accordance with one of claims 1 to 7.

10. A use of a carrier-drug conjugate in accordance with one of claims 1 to 7 for preparing a medical for inhibiting the growth of tumors in a mammal.

## Claims (Claims for the following Contracting State(s) : ES, GR)

1. A process for preparing the targeted derivatives 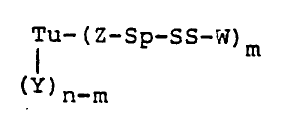 of compounds of formula CH₃SSS-W, wherein CH₃SSS-W is an antitumor antibiotic LL-E33288α₁, ^{Br}, α₁^{l}, α₂Br, α₂l, α₃^{Br}, α₃^{l}, α₄Br, β₁^{Br}, β₁^{l}, β₂^{Br}, β₂l, _{γ1} Br γ₁l, δ₁^{l}, BBM-1675, FR-900405, FR-900406, PD 114759, PD 115028, CL-1577A, CL-1577B, CL-1577D, CL-1577E, or CL-1724, comprising
reacting CH₃SSS-W with a compound of formula Q-Sp-SH, wherein Sp is a straight or branched-chain divalent (C₁-C₁₈) radical, divalent aryl or heteroaryl radical, divalent (C₃-C₁₈) cycloalkyl or heterocycloalkyl radical, divalent aryl- or heteroaryl-alkyl (C₁-C₁₈) radicals, divalent cycloalkyl- or heterocycloalkyl-alkyl (C₁-C₁₈) radical or divalent (C₂-Cₗ₈) unsaturated alkyl radical, and Q is halogen, amino, alkylamino, carboxyl, carboxaldehyde, hydroxy, lower alkyldicarboxyl anhydride, -CONHNH₂, -NHCONHNH₂, -NHCSNHNH₂, -ONH₂, or 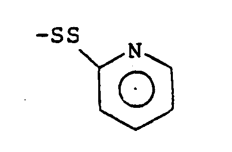 in acetonitrile in the presence of one equivalent of triethylamine and/or one equivalent of acetic acid at -10 to -30 _{°} C for 1-48 hours,
isolating the intermediate of formula Q-Sp-SS-W, wherein Q, Sp, and W are as hereinbefore defined, then
reacting the compound of formula Q-Sp-SS-W, wherein Sp and W are as hereinbefore defined and Q is halogen, amino, alkylamino, carboxyl, carboxaldehyde, hydroxy, or lower alkyldicarboxylic anhydride with a molecule of the formula Tu-(Y)ₙ wherein Tu is a mono- or polyclonal antibody, its fragments, its chemically or genetically manipulated counterparts, growth factors, or steroids; Y is a side-chain amino or carboxy functionality; n is 1-100, in aqueous buffer at a pH of between 6.5 and 9, at 4_{°} to 40 °C either directly or in the presence of a water-soluble carbodiimide, to generate the compound 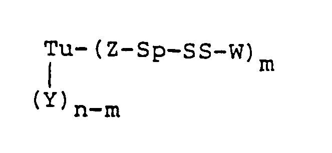 wherein Tu, Sp, W, n, and Y are as hereinbefore defined, m is 1-15 and Z is formed from covalent reaction of the groups Q and Y and is -CONH-, -NH-, 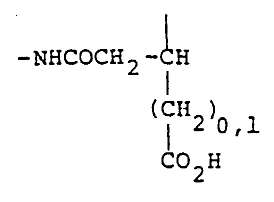 -N = CH-, or -C0₂-or
reacting the compound of formula Q-Sp-SS-W, wherein Sp and W are as hereinbefore defined and Q is a carboxylic acid, with N-hydroxysuccinimide, 2,3,5,6-tetrafluorophenol, pentafluorophenol, or 4-nitrophenol in the presence of a carboxyl activating agent such as a carbodiimide to generate a compound of formula Q-Sp-SS-W wherein Sp and W are as hereinbefore defined and Q is 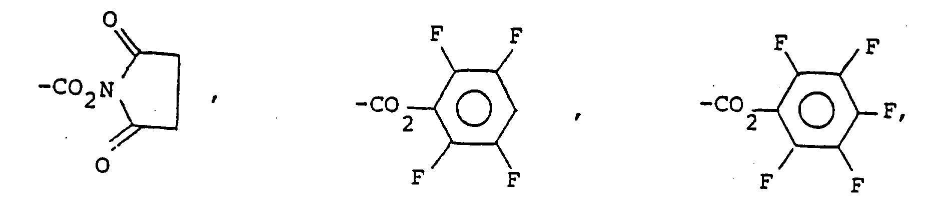 or 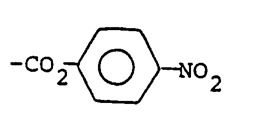 with a molecule of formula Tu-(Y)ₙ,
where Tu and n are as hereinbefore defined, and Y is a side-chain amino group, in an aqueous buffered solution at a pH between 6.5 and 9, at a temperature of between 4 _{°} and 40 _{°} C, inclusive, to generate compounds of the formula: 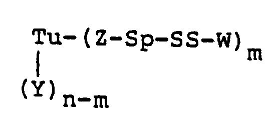 wherein Tu, Sp, Y, and n are as hereinbefore defined, m is 1-15, and Z is formed from covalent reaction between Q and Y and is defined as -CONH-or
reacting a compound of formula Q-Sp-SS-W, wherein Sp and W are as hereinbefore defined and Q is -CONHNH₂ with nitrous acid in aqueous acetonitrile to generate a compound of formula Q-Sp-SS-W, wherein Sp and W are as hereinbefore defined and Q is -CON₃ with a compound of formula Tu-(Y)ₙ, wherein Tu, Y, and n are as hereinabove defined to produce a compound of the formula 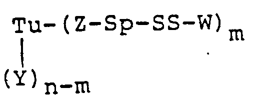 wherein Tu, Z, Sp, W, m, Y, and n are as hereinabove defined; or
reacting a compound of formula Q-Sp-SS-W wherein Sp and W are as hereinbefore defined and Q is hydroxy, with an alpha-haloacetic anhydride to produce a compound wherein Q is a-haloacetyloxy, and reacting the a-haloacetyloxy-Sp-SS-W or a compound of formula Q-Sp-SS-W, wherein Sp and W are as hereinbefore defined and Q is 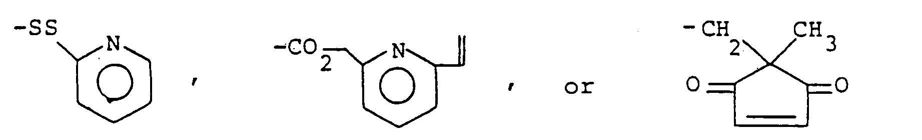 with a molecule of the formula Tu-(Y)ₙ wherein Tu is as hereinbefore defined, Y is a side-chain thiol of a protein, or an amidoalkylthio group introduced on an amine of Tu using reagents such as 3-(2-dithiopyridyl)propionic acid hydroxysuccinimide ester followed by reduction with an agent such as dithiothreitol, or an amidoalkylthio group introduced on an amine of Tu using 2-iminothiolane, and n is 1-10, under aqueous buffered conditions at a pH between 4.5 and 7, at a temperature between 4_{°} and 40 °C, inclusive, to produce a compound of formula: 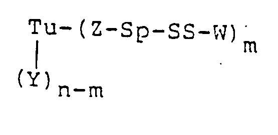 wherein Tu, Sp, W, and n are as hereinbefore defined, and Z is formed from covalent reaction of the groups Q and Y and Z is 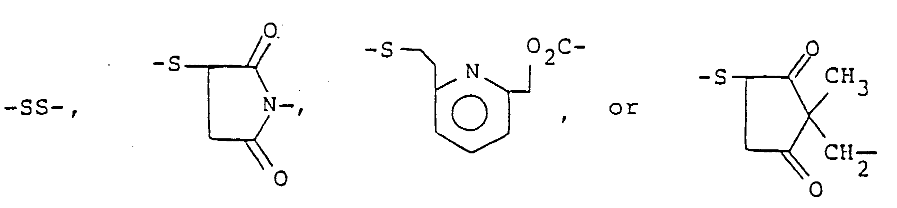 and n is 0.1 to 10; or
reacting a compound of the formula Q-Sp-SS-W wherein Sp and W are as hereinbefore defined and Q is -NH₂, -CONHNH₂, -NHCONHNH₂, -NHCSNHNH₂, or -ONH₂ with a molecule of formula Tu-(Y)ₙ wherein Tu is as hereinbefore defined, Y is an aldehyde generated from carbohydrate residues on Tu by oxidation in the presence of an alkaline earth periodate, in an aqueous buffer at a pH between 4.0 and 6.5, at 4 to 40 °C, inclusive, and n is 1 to 20 to generate a compound of formula: 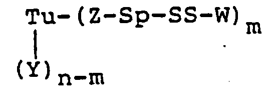 wherein Tu, Sp, W, Y, and n are as hereinbefore defined and Z is formed from the covalent reaction of Q and Y and is -ON = CH-, -N = CH-, -CONHN = CH-, -NHCONHN = CH-, or -NHCSNHN = CH-, and m is 0.1 to 15; or treating the compound immediately hereinabove of formula: 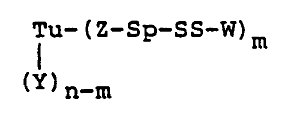 wherein Tu, Z, Sp, W, Y, n, and m are as immediately hereinabove defined with acetylhydrazine or tyrosine hydrazine in an aqueous buffer at a pH between 4.0 and 6.5, at 4° to 40 °C, inclusive, to generate a compound of formula: 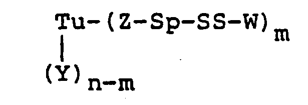 wherein Tu, Z, Sp, W, n, and m are as immediately hereinabove defined and Y is -CH = NNHCOCH₃ or 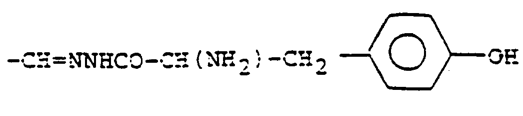 and
reacting this compound with sodium cyanoborohydride or sodium borohydride, in an aqueous buffer at a pH of 4.0 to 6.5, at a temperature of 4 to 40 °C, inclusive, to generate a compound of formula: 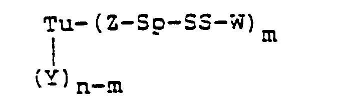 wherein Tu, Sp, W, m, and n are as hereinabove defined, Z is -NH-CH₂-, -CONHNHCH₂-, -NHCONHNHCH₂-, or -NHCSNHNHCH₂-, and Y is -CH₂NHNHCOCH₃ or

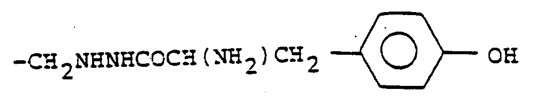

2. A process according to Claim 1 which produces a carrier-drug conjugate of the formula 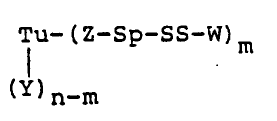 prepared from a compound of formula CH₃SSS-W wherein CH₃SSS-W is an antitumor antibiotic designated as LL-E33288α₁^{Br}, α₁^{l} α₂Br, α₂l, α₃^{Br}, α₃^{l}, α₄^{Br}, β₁^{Br}, β₁l_{,} β₂Br, β₂^{l}, γ₁ ^{Br}, γ₁l, δ₁l, BBM-₁₆₇₅, FR-900405, FR-900406, PD 114759, PD 115028, CL-1577A, CL-1577B, CL-1577D, CL-1577E, or CL-1724 comprising:
reacting CH₃SSS-W with a compound of general formula Q-Sp-SH, wherein Sp is a straight or branched-chain divalent (C₁-C₁₈) radical, divalent aryl or heteroaryl radical, divalent (C₃-C₁₈) cycloalkyl or heterocycloalkyl radical, divalent aryl- or heteroaryl-alkyl (C₁-C₁₈) radicals, divalent cycloalkyl- or heterocycloalkyl-alkyl (C₁-C₁₈) radical or divalent (C₂-C₁₈) unsaturated alkyl radical, and Q is, or can be subsequently converted to, halogen, amino, alkylamino, carboxyl, carboxaldehyde, hydroxy, thiol, a-haloacetyloxy, lower alkyldicarboxyl, -CONHNH₂, -NHCONHNH₂, -NHCSNHNH₂, -ONH₂, -CON3, 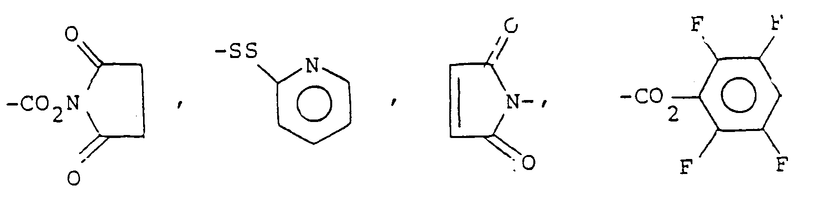 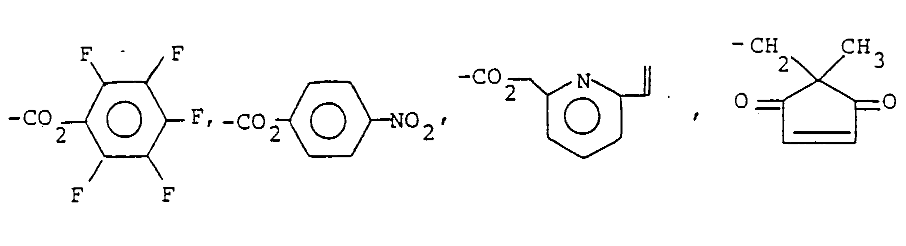 to produce an intermediate of formula Q-Sp-SS-W, wherein Q, Sp, and W are as hereinbefore defined,
reacting Q-Sp-SS-W with a molecule of the formula Tu-(Y)ₙ wherein Tu is defined as a mono- or polyclonal antibody, its fragments, its chemically or genetically manipulated counterparts, growth factors, or steroids; Y is a side-chain amino, carboxy, or thiol group of a protein, an aldehyde derived from carbohydrate residues, or an amidoalkylthio group; and n is an integer of from 1 to 100, to produce a compound of the formula: 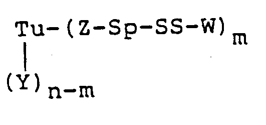 wherein Tu, Y, Sp, W, and n are as hereinbefore defined, and Z is formed from covalent reaction of the groups Q and Y directly or after subsequent reduction, and Z is -CONH-, -CONHN=CH-, -CONHNHCH₂-, -NHCONHN=CH-, -NHCONHNHCH₂-,-NHCSNHN = CH-, -NHCSNHNHCH₂-, -ON=CH- , -NH-, -NHCH₂-, -N=CH-, -C0₂-, -NHCH₂C0₂-, -SS-, 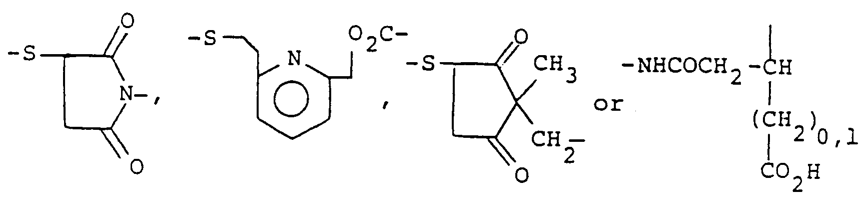 and m is 0.1 to 15.

3. A process according to Claim 1 which produces a protein-drug conjugate of the formula 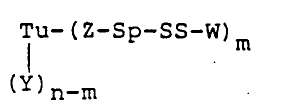 prepared from the antitumor antibiotic designated LL-E33288γ₁l (CH3SSS-W) having
a) ultraviolet spectrum as shown in Figure I;
b) a proton magnetic resonance spectrum as shown in Figure II; and
c) an infrared spectrum as shown in Figure III;
comprising:
displacing the dithiomethyl moiety with a compound of formula Q-Sp-SH, wherein Sp is straight or branched-chain divalent (C₂-C₅) radicals or divalent aryl- or heteroarylalkyl (C₂-C₅) radicals, and Q is, or can be subsequently converted to, carboxyl, lower alkyldicarboxylanhydride, -CONHNH₂, or 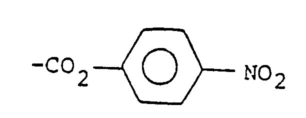 to produce an intermediate of general formula Q-Sp-SS-W, wherein Q, Sp, and W are as hereinbefore defined,
reacting Q-Sp-SS-W with a molecule of the formula Tu-(Y)ₙ wherein Tu is a monoclonal antibody which exhibits preferential reactivity with a human tumor-associated antigen, Y is a side-chain amino group on the antibody, or an aldehyde generated by oxidation of the carbohydrate groups of the antibody, and n is an integer of from 1 to 100, to produce a compound of the formula: 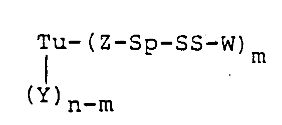 wherein Tu, Y, Sp, W, and n are as hereinbefore defined, and Z is formed from covalent reaction of the groups Q and Y directly or after subsequent reduction, and Z is -CONH-, -CONHN=CH-, -CONHNHCH₂-, or 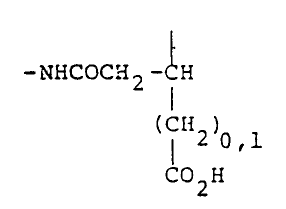 and m is 0.1 to 15.

4. A process according to Claim 1 wherein CH₃SSSW is LL-E33288_{γ1}^{l}, Q is the 4-nitrophenyl ester of a carboxyl group, Sp is -CH₂CH₂-, Tu is the monoclonal antibody CT-M-01, Y is -NH₂, Z is -CONH-, and m is 0.5 to 15.

5. A process according to Claim 1 wherein CH₃SSSW is LL-E33288_{γ1}l, Q is the hydroxysuccinimide ester of a carboxyl group, Sp is -CH₂CH₂-, Tu is the monoclonal antibody MAC-68, Y is -NH₂, Z is -CONH-, and m is 0.5 to 15.

6. A process according to Claim 1 wherein CH₃SSSW is LL-E33288_{γ1}^{l}, Q is -CONHNH₂, Sp is -CH₂CH₂-, Tu is the monoclonal antibody Lym 1, Y is -CHO, Z is -CONHNHCH₂-, and m is 0.1 to 10.

7. A process according to Claim 1 wherein CH₃SSSW is LL-E33288γ₁^{l}, Sp is 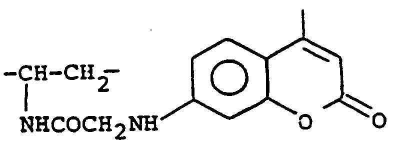 Tu is the monoclonal antibody B72.3, Y is -CHO, Z is -CONHNHCH₂-, and m is 0.1 to 10.

8. A process according to Claim 1 wherein CH₃SSSW is LL-E33288_{γ1}^{l}, Sp is 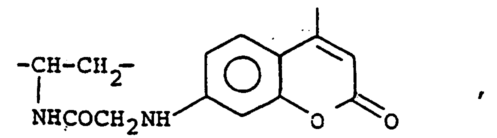 Tu is the monoclonal antibody Lym 2, Y is -CHO, Z is -CONHNHCH₂-, and m is 0.1 to 10.

## Patentansprüche (Patentansprüche für folgende(n) Vertragsstaat(en) : BE, CH, DE, FR, GB, IT, LI, LU, SE)

1. Träger-Arzneistoff-Konjugat der Formel 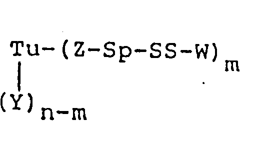 hergestellt aus einer Verbindung der Formel CH₃SSS-W, worin CH₃SSS-W ein Antitumor-Antibiotikum ist, das als LL-E33288α₁^{Br}, α₁^{l}, α₂^{Br}, α₂^{l}, a3^{Br}, α₃^{l}, α₄^{Br}, β₁^{Br}, β₁ β₂^{Br}, β2^{l}, γ₁^{Br}, γ l, δ₁^{l}, BBM-1675, FR-900405, FR-900406, PD 114759, PD 115028, CL-1577A, CL-1577B, CL-1577D, CL-1577E oder CL-1724 bezeichnet wird, umfassend: das Umsetzen von CH₃SSS-W mit einer Verbindung der allgemeinen Formel Q-Sp-SH, worin Sp ein geradkettiger oder verzweigtkettiger zweiwertiger (C₁-C₁₈)-Rest, zweiwertiger Aryl- oder Heteroarylrest, zweiwertiger (C₃-C₁₈)-Cycloalkyl- oder Heterocycloalkylrest, zweiwertige Aryl- oder Heteroaryl-(C₁-C₁₈)-alkylreste, ein zweiwertiger Cycloalkyl- oder Heterocycloalkyl-(C₁-C₁₈)-alkylrest oder zweiwertiger ungesättigter (C₂-C₁₈)-Alkylrest ist und Q Halogen, Amino, Alkylamino, Carboxyl, Carboxaldehyd, Hydroxy, Thiol, a-Halogenacetyloxy, Niederalkyldicarboxyl, -CONHNH₂, -NHCONHNH₂, -NHCSNHNH₂, -ONH₂, -CON₃, 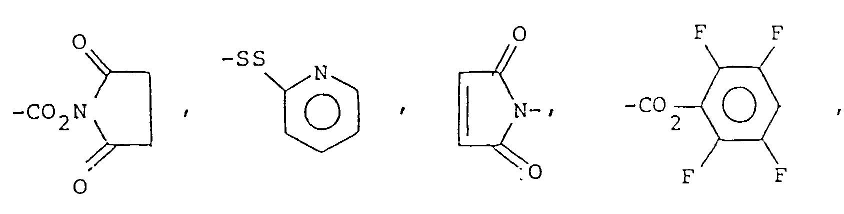 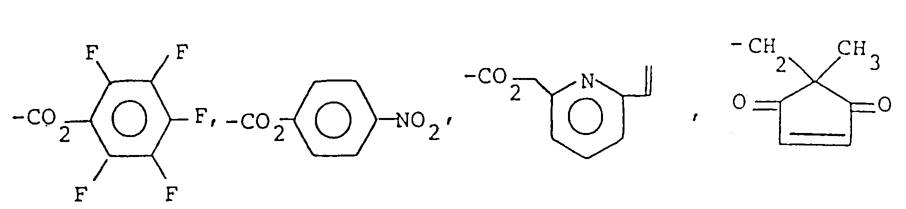 ist oder anschließend dazu umgewandelt werden kann, um ein Zwischenprodukt der Formel Q-Sp-SS-W zu erzeugen, worin Q, Sp und W wie vorstehend definiert sind,
das Umsetzen von Q-Sp-SS-W mit einem Molekül der Formel Tu-(Y)ₙ, worin Tu als mono- oder polyklonaler Antikörper, dessen Fragmente, dessen chemisch oder genetisch manipulierte Gegenstükke, Wachstumsfaktoren oder Steroide definiert ist; Y eine Amino-, Carboxy- oder Thiol-Seitenkettengruppe eines Proteins, ein von Kohlehydratresten abgeleiteter Aldehyd oder eine Amidoalkylthio-Gruppe ist; und n eine ganze Zahl von 1 bis 100 ist, um eine Verbindung der Formel: 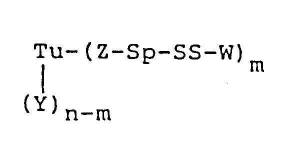 zu erzeugen, worin Tu, Y, Sp, W und n wie vorstehend definiert sind und Z aus einer kovalenten Reaktion der Gruppen Q und Y direkt oder nach anschließender Reduktion gebildet ist und Z -CONH-, -CONHN=CH-, -CONHNHCH₂-, -NHCONHN=CH-, -NHCONHNHCH₂-, -NHCSNHN=CH-, -NHCSNHNHCH₂-, -ON=CH-, -NH-, -NHCH₂-, -N=CH-, -C0₂-, -NHCH₂C0₂-, -SS-, 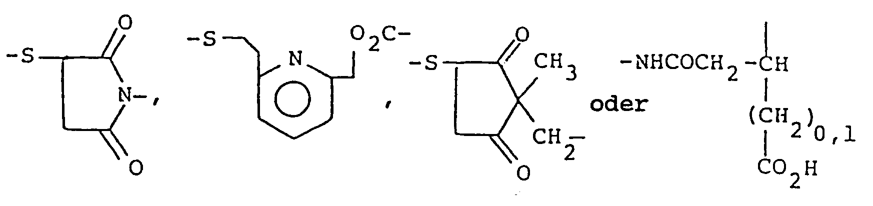 ist und m 0,1 bis 15 ist.

2. Protein-Arzneistoff-Konjugat der Formel 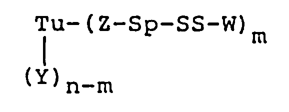 hergestellt aus dem Antitumor-Antibiotikum, das als LL-E33288γ₁l (CH3SSS-W) bezeichnet wird mit
a) einem Ultraviolettspektrum wie in Figur I gezeigt;
b) einem magnetischen Protonenresonanzspektrum wie in Figur 11 gezeigt; und
c) einem Infrarotspektrum wie in Figur III gezeigt;
umfassend:
das Verdrängen der Dithiomethyl-Einheit durch eine Verbindung der Formel Q-Sp-SH, worin es sich bei Sp um geradkettige oder verzweigtkettige zweiwertige (C₂-C₅)-Reste oder zweiwertige Aryl- oder Heteroaryl-(C₂-Cₛ)-alkylreste handelt und Q Carboxyl-, Niederalkyldicarboxylanhydrid, -CONHNH₂ oder 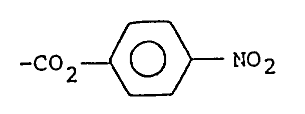 ist oder dazu umgewandelt werden kann, um ein Zwischenprodukt der allgemeinen Formel Q-Sp-SS-W zu erzeugen, worin Q, Sp und W wie vorstehend definiert sind,
das Umsetzen von Q-Sp-SS-W mit einem Molekül der Formel Tu-(Y)ₙ, worin Tu ein monoklonaler Antikörper ist, der eine bevorzugte Reaktivität mit einem menschlichen Tumorassoziierten Antigen aufweist, Y eine Amino-Seitenkettengruppe an dem Antikörper oder ein Aldehyd ist, der durch Oxidation der Kohlehydrat-Gruppen des Antikörpers erzeugt wird, und n eine ganze Zahl von 1 bis 100 ist, um eine Verbindung der Formel: 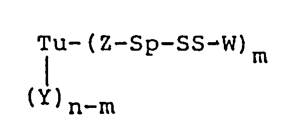 zu erzeugen, worin Tu, Y, Sp, W und n wie vorstehend definiert sind und Z aus einer kovalenten Reaktion der Gruppen Q und Y direkt oder nach anschließender Reduktion gebildet ist und Z -CONH-, -CONHN=CH-, -CONHNHCH₂- oder 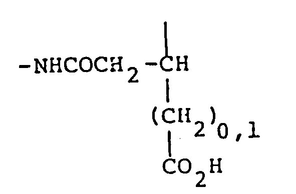 ist und m 0,1 bis 15 ist.

3. Verbindung nach Anspruch 1, worin CH₃SSSW LL-E33288γ₁l ist, Q der 4-Nitrophenylester einer Carboxylgruppe ist, Sp -CH₂CH₂- ist, Tu der monoklonale Antikörper CT-M-01 ist, Y -NH₂ ist, Z -CONH- ist und m 0,5 bis 15 ist.

4. Verbindung nach Anspruch 1, worin CH₃SSSW LL-E33288γ₁l ist, Q der Hydroxysuccinimid-Ester einer Carboxylgruppe ist, Sp -CH₂CH₂- ist, Tu der monoklonale Antikörper MAC-68 ist, Y -NH₂ ist, Z -CONHist und m 0,5 bis 15 ist.

5. Verbindung nach Anspruch 1, worin CH₃SSSW LL-E33288γ₁l ist, Q -CONHNH₂ ist, Sp -CH₂CH₂- ist, Tu der monoklonale Antikörper Lym 1 ist, Y -CHO ist, Z -CONHNHCH₂- ist und m 0,1 bis 10 ist.

6. Verbindung nach Anspruch 1, worin CH₃SSSW LL-E33288γ₁l ist, Sp 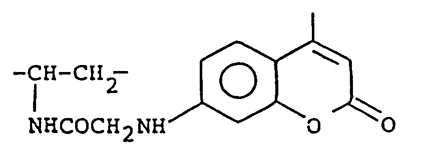 ist, Tu der monoklonale Antikörper B72.3 ist, Y -CHO ist, Z -CONHNHCH₂- ist und m 0,1 bis 10 ist.

7. Verbindung nach Anspruch 1, worin CH₃SSSW LL-E33288γ₁l ist, Sp 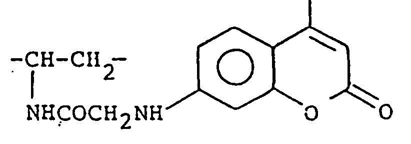 ist, Tu der monoklonale Antikörper Lym 2 ist, Y -CHO ist, Z -CONHNHCH₂- ist und m 0,1 bis 10 ist.

8. Verfahren zur Herstellung der Ziel-Derivate 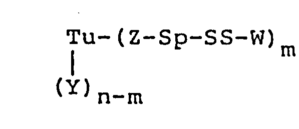 von Verbindungen der Formel CH₃SSS-W, worin CH₃SSS-W ein Antitumor-Antibiotikum LL-E33288α₁ ^{Br}, α₁^{l}, α₃^{Br}, α₃^{l}, α₄^{Br}, β₁^{Br}, β₁^{l}, β₂^{Br}, β₂^{l}, γ₁^{Br}, γ₁^{l}, δ₁^{l}, BBM-1675, FR-900405, FR-900406, PD 114759, PD 115028, CL-1577A, CL-1577B, CL-1577D, CL-1577E oder CL-1724 ist, umfassend
das Umsetzen von CH₃SSS-W mit einer Verbindung der Formel Q-Sp-SH, worin Sp ein geradkettiger oder verzweigtkettiger zweiwertiger (C₁-C₁₈)-Rest, zweiwertiger Aryl- oder Heteroarylrest, zweiwertiger (C₃-C₁₈)-Cycloalkyl- oder Heterocycloalkylrest, zweiwertige Aryl- oder Heteroaryl-(C₁-C₁₈)-alkylreste, ein zweiwertiger Cycloalkyl- oder Heterocycloalkyl-(C₁-C₁₈)-alkylrest oder ungesättigter zweiwertiger (C₂-C₁₈)-Alkylrest ist und Q Halogen, Amino, Alkylamino, Carboxyl, Carboxaldehyd, Hydroxy, Niederalkyldicarboxylanhydrid, -CONHNH₂, -NHCONHNH₂, -NHCSNHNH₂, -ONH₂ oder 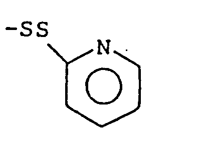 ist, in Acetonitril in Gegenwart eines Äquivalents Triethylamin und/oder eines Äquivalents Essigsäure bei -10 bis -30 °C über 1-48 Stunden,
das Isolieren des Zwischenprodukts der Formel Q-Sp-SS-W, worin Q, Sp und W wie vorstehend definiert sind, dann das Umsetzen der Verbindung der Formel Q-Sp-SS-W, worin Sp und W wie vorstehend definiert sind und Q Halogen, Amino, Alkylamino, Carboxyl, Carboxaldehyd, Hydroxy oder Niederalkyldicarbonsäureanhydrid ist, mit einem Molekül der Formel Tu-(Y)ₙ, worin Tu für einen mono-oder polyklonalen Antikörper, dessen Fragmente, dessen chemisch oder genetisch manipulierte Gegenstücke, Wachstumsfaktoren oder Steroide steht; Y eine Amino- oder Carboxy-Seitenkettenfunktionalität ist; n 1-100 ist, bei 4 bis 40 °C in wäßrigem Puffer bei einem pH zwischen 6,5 und 9, entweder direkt oder in Gegenwart eines wasserlöslichen Carbodiimids, um die Verbindung 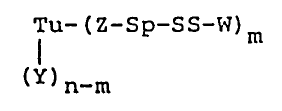 zu erzeugen, worin Tu, Sp, W, n und Y wie vorstehend definiert sind, m 1-15 ist und Z aus einer kovalenten Reaktion der Gruppen Q und Y gebildet ist und -CONH-, -NH-, 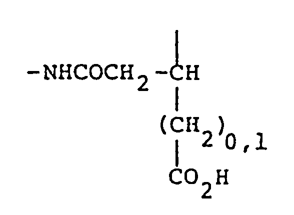 -N = CH- oder -C0₂- ist, oder
das Umsetzen der Verbindung der Formel Q-Sp-SS-W, worin Sp und W wie vorstehend definiert sind und Q eine Carbonsäure ist, mit N-Hydroxysuccinimid, 2,3,5,6-Tetrafluorphenol, Pentafluorphenol oder 4-Nitrophenol in Gegenwart eines Carboxyl-aktivierenden Mittels, wie z.B. Carbodiimid, um eine Verbindung der Formel Q-Sp-SS-W zu erzeugen, worin Sp und W wie vorstehend definiert sind und Q 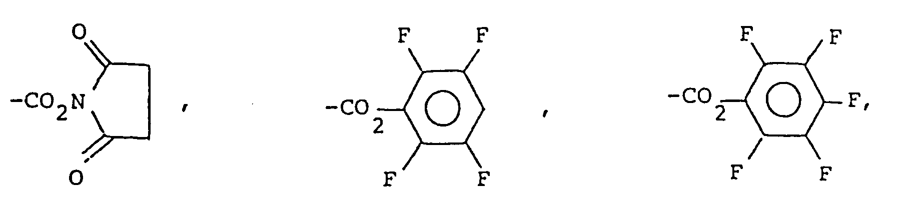 oder 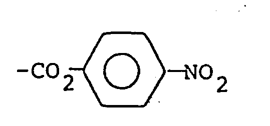 ist, mit einem Molekül der Formel Tu-(Y)ₙ,
worin Tu und n wie vorstehend definiert sind und Y eine Amino-Seitenkettengruppe ist, bei einer Temperatur zwischen 4 und 40 °C einschließlich in einer wäßrigen gepufferten Lösung bei einem pH zwischen 6,5 und 9, um Verbindungen der Formel: 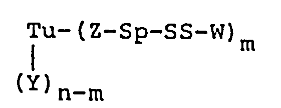 zu erzeugen, worin Tu, Sp, Y und n wie vorstehend definiert sind, m 1-15 ist und Z aus einer kovalenten Reaktion zwischen Q und Y gebildet ist und als -CONH- definiert ist oder
das Umsetzen einer Verbindung der Formel Q-Sp-SS-W, worin Sp und W wie vorstehend definiert sind und Q -CONHNH₂ ist, mit salpetriger Säure in wäßrigem Acetonitril, um eine Verbindung der Formel Q-Sp-SS-W zu erzeugen, worin Sp und W wie vorstehend definiert sind und Q -CON₃ ist, mit einer Verbindung der Formel Tu-(Y)ₙ, worin Tu, Y und n wie vorstehend definiert sind, um eine Verbindung der Formel 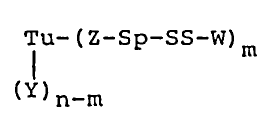 zu erzeugen, worin Tu, Z, Sp, W, m, Y und n wie vorstehend definiert sind; oder
das Umsetzen einer Verbindung der Formel Q-Sp-SS-W, worin Sp und W wie vorstehend definiert sind und Q Hydroxy ist, mit einem alpha-Halogenessigsäureanhydrid, um eine Verbindung zu erzeugen, worin Q a-Halogenacetyloxy ist, und das Umsetzen des a-Halogenacetyloxy-Sp-SS-W oder einer Verbindung der Formel Q-Sp-SS-W, worin Sp und W wie vorstehend definiert sind und Q 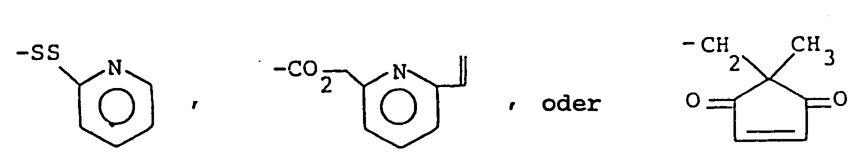 ist, mit einem Molekül der Formel Tu-(Y)ₙ, worin Tu wie vorstehend definiert ist, Y ein Seitenketten-Thiol eines Proteins oder eine Amidoalkylthio-Gruppe, die an einem Amin von Tu unter Verwendung von Reagenzien, wie beispielsweise 3-(2-Dithiopyridyl)propionsäurehydroxysuccinimidester, gefolgt von der Reduktion mit einem Mittel, wie beispielsweise Dithiothreitol, eingeführt wurde, oder eine Amidoalkylthio-Gruppe, die an einem Amin von Tu unter Verwendung von 2-Iminothiolan eingeführt wurde, ist und n 1-10 ist, bei einer Temperatur zwischen 4 und 40 °C einschließlich unter wäßrigen gepufferten Bedingungen bei einem pH zwischen 4,5 und 7, um eine Verbindung der Formel: 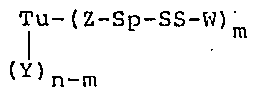 zu erzeugen, worin Tu, Sp, W und n wie vorstehend definiert sind und Z aus einer kovalenten Reaktion der Gruppen Q und Y gebildet ist und Z 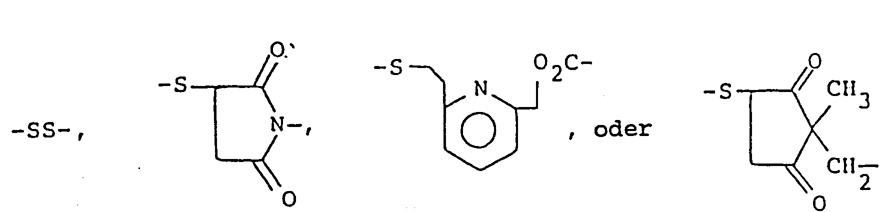 ist und n 0,1 bis 10 ist; oder
das Umsetzen einer Verbindung der Formel Q-Sp-SS-W, worin Sp und W wie vorstehend definiert sind und Q -NH₂, -CONHNH₂, -NHCONHNH₂, -NHCSNHNH₂ oder -ONH₂ ist, mit einem Molekül der Formel Tu-(Y)ₙ, worin Tu wie vorstehend definiert ist, Y ein Aldehyd ist, der aus Kohlehydratresten an Tu durch Oxidation in Gegenwart eines Erdalkaliperiodats erzeugt wurde, bei 4 bis 40 °C einschließlich in einem wäßrigen Puffer bei einem pH zwischen 4,0 und 6,5, und n 1 bis 20 ist, um eine Verbindung der Formel 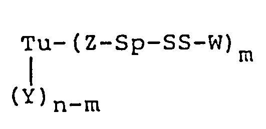 zu erzeugen, worin Tu, Sp, W, Y und n wie vorstehend definiert sind und Z aus der kovalenten Reaktion von Q und Y gebildet ist und -ON = CH-, -N = CH-, -CONHN = CH-, -NHCONHN = CH- oder -NHCSNHN=CH- ist und m 0,1 bis 15 ist; oder das Behandeln der unmittelbar oben aufgeführten Verbindung der Formel: 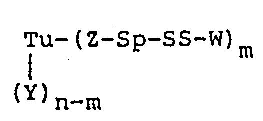 worin Tu, Z, Sp, W, Y, n und m wie unmittelbar oben definiert sind, mit Acetylhydrazin oder Tyrosinhydrazin bei 4 bis 40 °C einschließlich in einem wäßrigen Puffer bei einem pH zwischen 4,0 und 6,5, um eine Verbindung der Formel: 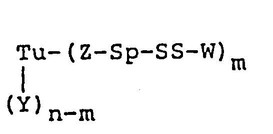 zu erzeugen, worin Tu, Z, Sp, W, n und m wie unmittelbar oben definiert sind und Y-CH = NNHCOCH₃ oder 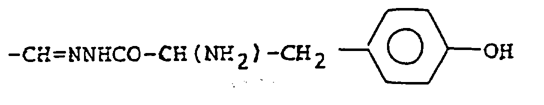 ist, und
das Umsetzen dieser Verbindung mit Natriumcyanborhydrid oder Natriumborhydrid bei einer Temperatur von 4° bis 40 °C einschließlich in einem wäßrigen Puffer bei einem pH von 4,0 bis 6,5, um eine Verbindung der Formel: 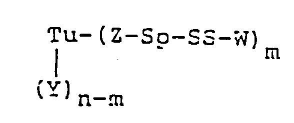 zu erzeugen, worin Tu, Sp, W, m und n wie oben definiert sind, Z -NH-CH₂-, -CONHNHCH₂-, -NHCONHNHCH₂- oder -NHCSNHNHCH₂- ist und Y -CH2NHNHCOCH3 oder 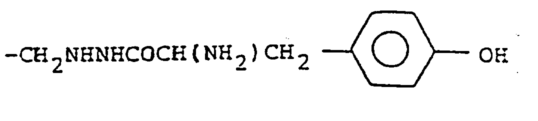 ist.

9. Pharmazeutische Zusammensetzung, enthaltend ein Träger-Arzneistoff-Konjugat nach einem der Ansprüche 1 bis 7.

10. Verwendung eines Träger-Arzneistoff-Konjugats nach einem der Ansprüche 1 bis 7 bei der Herstellung eines Medikaments zum Hemmen des Wachstums von Tumoren in einem Säuger.

## Patentansprüche (Patentansprüche für folgende(n) Vertragsstaat(en) : ES, GR)

1. Verfahren zur Herstellung der Ziel-Derivate 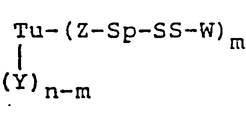 von Verbindungen der Formel CH₃SSS-W, worin CH₃SSS-W ein Antitumor-Antibiotikum LL-E33288α₁ Br, α₁ α₂^{Br}, α₂^{l}, α₃^{Br},α₃l, α₄Br, β₁Br, β₁l, β₂^{Br}, β₂^{l} γ₁^{Br}, γ₁^{l}, δ₁^{l}, BBM-1675, FR-900405, FR-900406, PD 114759, PD 115028, CL-1577A, CL-1577B, CL-1577D, CL-1577E oder CL-1724 ist, umfassend
das Umsetzen von CH₃SSS-W mit einer Verbindung der Formel Q-Sp-SH, worin Sp ein geradkettiger oder verzweigtkettiger zweiwertiger (C₁-C₁₈)-Rest, zweiwertiger Aryl- oder Heteroarylrest, zweiwertiger (C₃-C₁₈)-Cycloalkyl- oder Heterocycloalkylrest, zweiwertige Aryl- oder Heteroaryl-(C₁-C₁₈)-alkylreste, ein zweiwertiger Cycloalkyl- oder Heterocycloalkyl-(C₁-C₁₈)-alkylrest oder ungesättigter zweiwertiger (C₂-C₁₈)-Alkylrest ist und Q Halogen, Amino, Alkylamino, Carboxyl, Carboxaldehyd, Hydroxy, Niederalkyldicarboxylanhydrid, -CONHNH₂, -NHCONHNH₂, -NHCSNHNH₂, -ONH₂ oder 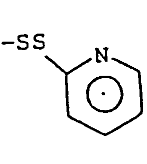 ist, in Acetonitril in Gegenwart eines Äquivalents Triethylamin und/oder eines Äquivalents Essigsäure bei -10 bis -30 °C über 1-48 Stunden,
das Isolieren des Zwischenprodukts der Formel Q-Sp-SS-W, worin Q, Sp und W wie vorstehend definiert sind, dann das Umsetzen der Verbindung der Formel Q-Sp-SS-W, worin Sp und W wie vorstehend definiert sind und Q Halogen, Amino, Alkylamino, Carboxyl, Carboxaldehyd, Hydroxy oder Niederalkyldicarbonsäureanhydrid ist, mit einem Molekül der Formel Tu-(Y)ₙ, worin Tu für einen mono-oder polyklonalen Antikörper, dessen Fragmente, dessen chemisch oder genetisch manipulierte Gegenstücke, Wachstumsfaktoren oder Steroide steht; Y eine Amino- oder Carboxy-Seitenkettenfunktionalität ist; n 1-100 ist, bei 4 bis 40 °C in wäßrigem Puffer bei einem pH zwischen 6,5 und 9, entweder direkt oder in Gegenwart eines wasserlöslichen Carbodiimids, um die Verbindung 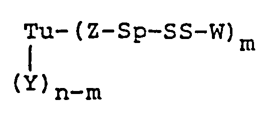 zu erzeugen, worin Tu, Sp, W, n und Y wie vorstehend definiert sind, m 1-15 ist und Z aus einer kovalenten Reaktion der Gruppen Q und Y gebildet ist und -CONH-, -NH-, 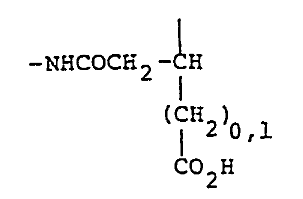 -N = CH- oder -C0₂- ist, oder
das Umsetzen der Verbindung der Formel Q-Sp-SS-W, worin Sp und W wie vorstehend definiert sind und Q eine Carbonsäure ist, mit N-Hydroxysuccinimid, 2,3,5,6-Tetrafluorphenol, Pentafluorphenol oder 4-Nitrophenol in Gegenwart eines Carboxyl-aktivierenden Mittels, wie z.B. Carbodiimid, um eine Verbindung der Formel Q-Sp-SS-W zu erzeugen, worin Sp und W wie vorstehend definiert sind und Q 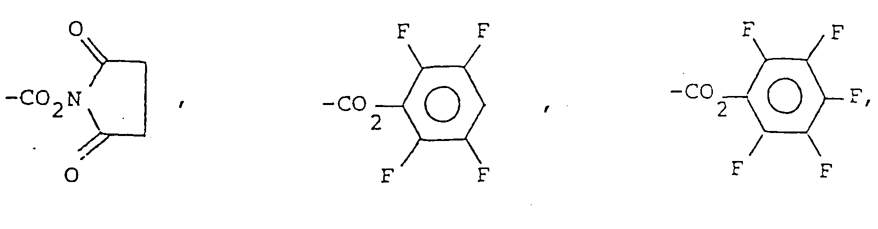 oder 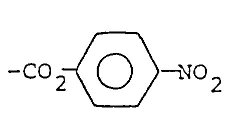 ist, mit einem Molekül der Formel Tu-(Y)ₙ,
worin Tu und n wie vorstehend definiert sind und Y eine Amino-Seitenkettengruppe ist, bei einer Temperatur zwischen 4 und 40 °C einschließlich in einer wäßrigen gepufferten Lösung bei einem pH zwischen 6,5 und 9, um Verbindungen der Formel: 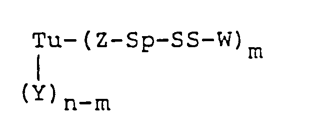 zu erzeugen, worin Tu, Sp, Y und n wie vorstehend definiert sind, m 1-15 ist und Z aus einer kovalenten Reaktion zwischen Q und Y gebildet ist und als -CONH- definiert ist oder
das Umsetzen einer Verbindung der Formel Q-Sp-SS-W, worin Sp und W wie vorstehend definiert sind und Q -CONHNH₂ ist, mit salpetriger Säure in wäßrigem Acetonitril, um eine Verbindung der Formel Q-Sp-SS-W zu erzeugen, worin Sp und W wie vorstehend definiert sind und Q -CON₃ ist, mit einer Verbindung der Formel Tu-(Y)ₙ, worin Tu, Y und n wie vorstehend definiert sind, um eine Verbindung der Formel 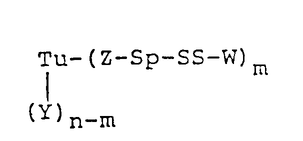 zu erzeugen, worin Tu, Z, Sp, W, m, Y und n wie vorstehend definiert sind; oder
das Umsetzen einer Verbindung der Formel Q-Sp-SS-W, worin Sp und W wie vorstehend definiert sind und Q Hydroxy ist, mit einem alpha-Halogenessigsäureanhydrid, um eine Verbindung zu erzeugen, worin Q a-Halogenacetyloxy ist, und das Umsetzen des a-Halogenacetyloxy-Sp-SS-W oder einer Verbindung der Formel Q-Sp-SS-W, worin Sp und W wie vorstehend definiert sind und Q 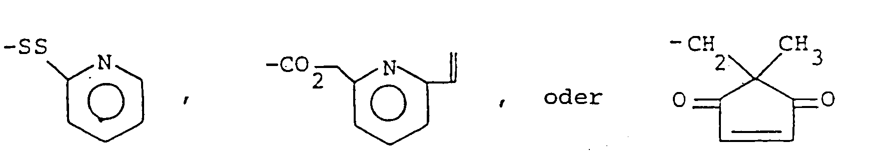 ist, mit einem Molekül der Formel Tu-(Y)ₙ, worin Tu wie vorstehend definiert ist, Y ein Seitenketten-Thiol eines Proteins oder eine Amidoalkylthio-Gruppe, die an einem Amin von Tu unter Verwendung von Reagenzien, wie beispielsweise 3-(2-Dithiopyridyl)propionsäurehydroxysuccinimidester, gefolgt von der Reduktion mit einem Mittel, wie beispielsweise Dithiothreitol, eingeführt wurde, oder eine Amidoalkylthio-Gruppe, die an einem Amin von Tu unter Verwendung von 2-Iminothiolan eingeführt wurde, ist und n 1-10 ist, bei einer Temperatur zwischen 4 und 40 °C einschließlich unter wäßrigen gepufferten Bedingungen bei einem pH zwischen 4,5 und 7, um eine Verbindung der Formel: 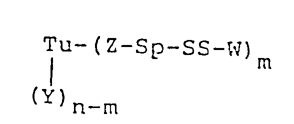 zu erzeugen, worin Tu, Sp, W und n wie vorstehend definiert sind und Z aus einer kovalenten Reaktion der Gruppen Q und Y gebildet ist und Z 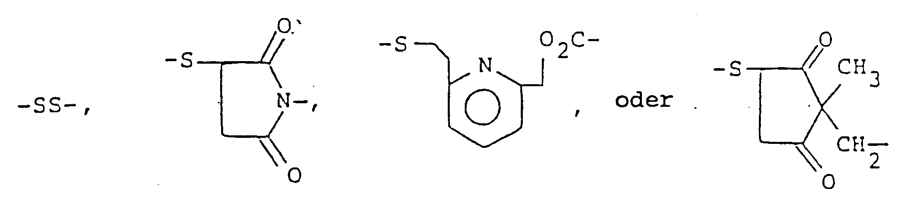 ist und n 0,1 bis 10 ist; oder
das Umsetzen einer Verbindung der Formel Q-Sp-SS-W, worin Sp und W wie vorstehend definiert sind und Q -NH₂, -CONHNH₂, -NHCONHNH₂, -NHCSNHNH₂ oder -ONH₂ ist, mit einem Molekül der Formel Tu-(Y)ₙ, worin Tu wie vorstehend definiert ist, Y ein Aldehyd ist, der aus Kohlehydratresten an Tu durch Oxidation in Gegenwart eines Erdalkaliperiodats erzeugt wurde, bei 4 bis 40 °C einschließlich in einem wäßrigen Puffer bei einem pH zwischen 4,0 und 6,5, und n 1 bis 20 ist, um eine Verbindung der Formel: 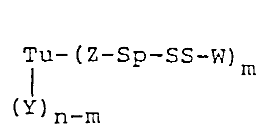 zu erzeugen, worin Tu, Sp, W, Y und n wie vorstehend definiert sind und Z aus der kovalenten Reaktion von Q und Y gebildet ist und -ON = CH-, -N = CH-, -CONHN = CH-, -NHCONHN = CH- oder -NHCSNHN=CH- ist und m 0,1 bis 15 ist; oder das Behandeln der unmittelbar oben aufgeführten Verbindung der Formel: 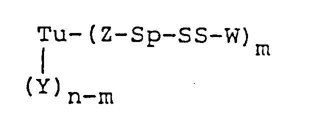 worin Tu, Z, Sp, W, Y, n und m wie unmittelbar oben definiert sind, mit Acetylhydrazin oder Tyrosinhydrazin bei 4° bis 40 °C einschließlich in einem wäßrigen Puffer bei einem pH zwischen 4,0 und 6,5, um eine Verbindung der Formel: 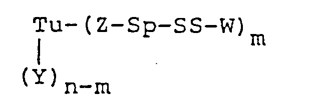 zu erzeugen, worin Tu, Z, Sp, W, n und m wie unmittelbar oben definiert sind und Y-CH = NNHCOCH₃ oder 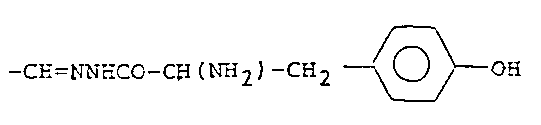 ist, und
das Umsetzen dieser Verbindung mit Natriumcyanborhydrid oder Natriumborhydrid bei einer Temperatur von 4° bis 40 °C einschließlich in einem wäßrigen Puffer bei einem pH von 4,0 bis 6,5, um eine Verbindung der Formel: 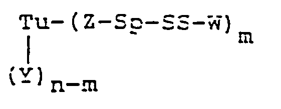 zu erzeugen, worin Tu, Sp, W, m und n wie oben definiert sind, Z -NH-CH₂-, -CONHNHCH₂-, -NHCONHNHCH₂- oder -NHCSNHNHCH₂- ist und Y -CH2NHNHCOCH3 oder 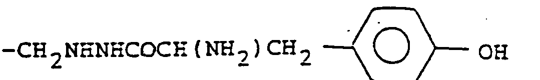 ist.

2. Verfahren nach Anspruch 1, das ein Träger-Arzneistoff-Konjugat der Formel 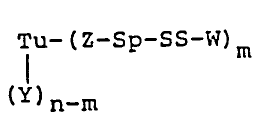 erzeugt, das hergestellt ist aus einer Verbindung der Formel CH₃SSS-W, worin CH₃SSS-W ein Antitumor-Antibiotikum ist, das als LL-E33288α₁, ^{Br}, α₁^{l}, α₂^{Br}, α₂l, α₃^{Br}, α₃l, α₄^{Br}, β₁ ^{Br}, β₁^{l}, β₂^{Br}, β₂^{l}, γ₁ ^{B}_{'}, γ₁^{l}, δ₁^{l}, BBM-1675, FR-900405, FR-900406, PD 114759, PD 115028, CL-1577A, CL-1577B, CL-1577D, CL-1577E oder CL-1724 bezeichnet wird, umfassend:
das Umsetzen von CH₃SSS-W mit einer Verbindung der allgemeinen Formel Q-Sp-SH, worin Sp ein geradkettiger oder verzweigtkettiger zweiwertiger (C₁-C₁₈)-Rest, zweiwertiger Aryl- oder Heteroarylrest, zweiwertiger (C₃-C_{1 8})-Cycloalkyl- oder Heterocycloalkylrest, zweiwertige Aryl- oder Heteroaryl-(C₁ -C ₁₈)-alkylreste, ein zweiwertiger Cycloalkyl- oder Heterocycloalkyl-(C₁ -C ₁₈)-alkylrest oder zweiwertiger ungesättigter (C₂-C₁₈)-Alkylrest ist und Q Halogen, Amino, Alkylamino, Carboxyl, Carboxaldehyd, Hydroxy, Thiol, a-Halogenacetyloxy, Niederalkyldicarboxyl, -CONHNH₂, -NHCONHNH₂, -NHCSNHNH₂, -ONH₂, -CON₃, 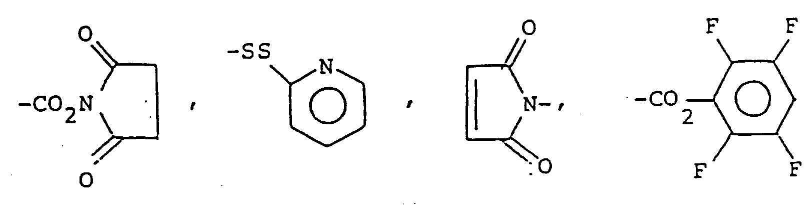 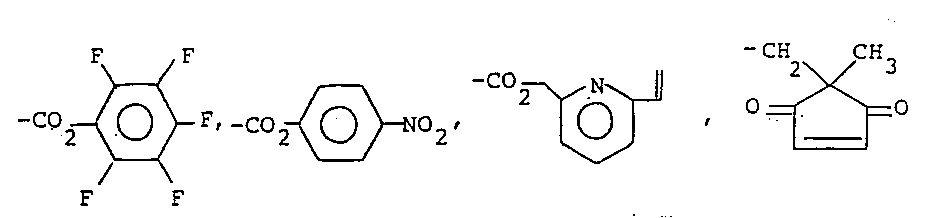 ist oder anschließend dazu umgewandelt werden kann, um ein Zwischenprodukt der Formel Q-Sp-SS-W zu erzeugen, worin Q, Sp und W wie vorstehend definiert sind,
das Umsetzen von Q-Sp-SS-W mit einem Molekül der Formel Tu-(Y)ₙ, worin Tu als mono- oder polyklonaler Antikörper, dessen Fragmente, dessen chemisch oder genetisch manipulierte Gegenstükke, Wachstumsfaktoren oder Steroide definiert ist; Y eine Amino-, Carboxy- oder Thiol-Seitenkettengruppe eines Proteins, ein von Kohlehydratresten abgeleiteter Aldehyd oder eine Amidoalkylthio-Gruppe ist; und n eine ganze Zahl von 1 bis 100 ist, um eine Verbindung der Formel: 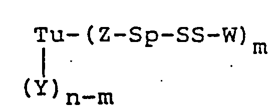 zu erzeugen, worin Tu, Y, Sp, W und n wie vorstehend definiert sind und Z aus einer kovalenten Reaktion der Gruppen Q und Y direkt oder nach anschließender Reduktion gebildet ist und Z -CONH-, -CONHN=CH-, -CONHNHCH₂-, -NHCONHN=CH-, -NHCONHNHCH₂-, -NHCSNHN=CH-, -NHCSNHNHCH₂-, -ON=CH-, -NH-, -NHCH₂-, -N=CH-, -C0₂-, -NHCH₂C0₂-, -SS-, 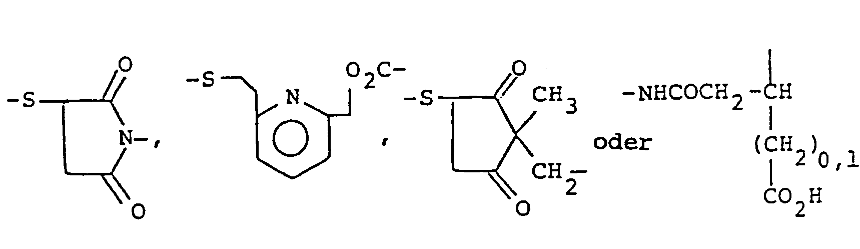 ist und m 0,1 bis 15 ist.

3. Verfahren nach Aspruch 1, das ein Protein-Arzneistoff-Konjugat der Formel 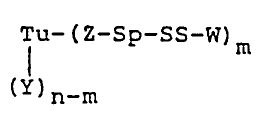 erzeugt, das hergestellt ist aus dem Antitumor-Antibiotikum, das als LL-E33288γ₁l (CH₃SSS-W) bezeichnet wird mit
a) einem Ultraviolettspektrum wie in Figur I gezeigt;
b) einem magnetischen Protonenresonanzspektrum wie in Figur 11 gezeigt; und
c) einem Infrarotspektrum wie in Figur III gezeigt;
umfassend:
das Verdrängen der Dithiomethyl-Einheit durch eine Verbindung der Formel Q-Sp-SH, worin es sich bei Sp um geradkettige oder verzweigtkettige zweiwertige (C₂-C₅)-Reste oder zweiwertige Aryl- oder Heteroaryl-(C₂-Cₛ)-alkylreste handelt und Q Carboxyl-, Niederalkyldicarboxylanhydrid, -CONHNH₂ oder 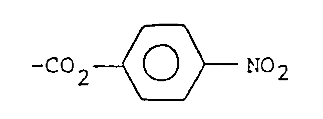 ist oder dazu umgewandelt werden kann, um ein Zwischenprodukt der allgemeinen Formel Q-Sp-SS-W zu erzeugen, worin Q, Sp und W wie vorstehend definiert sind,
das Umsetzen von Q-Sp-SS-W mit einem Molekül der Formel Tu-(Y)ₙ, worin Tu ein monoklonaler Antikörper ist, der eine bevorzugte Reaktivität mit einem menschlichen Tumorassoziierten Antigen aufweist, Y eine Amino-Seitenkettengruppe an dem Antikörper oder ein Aldehyd ist, der durch Oxidation der Kohlehydrat-Gruppen des Antikörpers erzeugt wird, und n eine ganze Zahl von 1 bis 100 ist, um eine Verbindung der Formel: 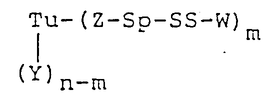 zu erzeugen, worin Tu, Y, Sp, W und n wie vorstehend definiert sind und Z aus einer kovalenten Reaktion der Gruppen Q und Y direkt oder nach anschließender Reduktion gebildet ist und Z -CONH-, -CONHN=CH-, -CONHNHCH₂- oder 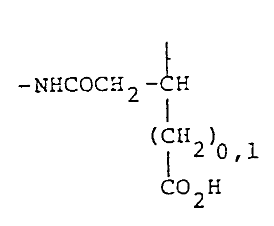 ist und m 0,1 bis 15 ist.

4. Verfahren nach Anspruch 1, worin CH₃SSSW LL-E33288γ₁l ist, Q der 4-Nitrophenylester einer Carboxylgruppe ist, Sp -CH₂CH₂- ist, Tu der monoklonale Antikörper CT-M-01 ist, Y -NH₂ ist, Z -CONH- ist und m 0,5 bis 15 ist.

5. Verfahren nach Anspruch 1, worin CH₃SSSW LL-E33288_{γ1}l ist, Q der Hydroxysuccinimid-Ester einer Carboxylgruppe ist, Sp -CH₂CH₂- ist, Tu der monoklonale Antikörper MAC-68 ist, Y -NH₂ ist, Z -CONHist und m 0,5 bis 15 ist.

6. Verfahren nach Anspruch 1, worin CH₃SSSW LL-E33288_{γ1}l ist, Q -CONHNH₂ ist, Sp -CH₂CH₂- ist, Tu der monoklonale Antikörper Lym 1 ist, Y -CHO ist, Z -CONHNHCH₂- ist und m 0,1 bis 10 ist.

7. Verfahren nach Anspruch 1, worin CH₃SSSW LL-E33288_{γ1}l ist, Sp 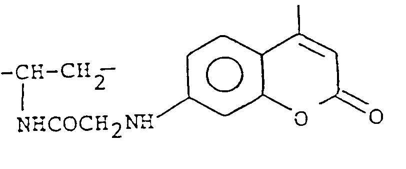 ist, Tu der monoklonale Antikörper B72.3 ist, Y -CHO ist, Z -CONHNHCH₂- ist und m 0,1 bis 10 ist.

8. Verfahren nach Anspruch 1, worin CH₃SSSW LL-E33288_{γ1}l ist, Sp 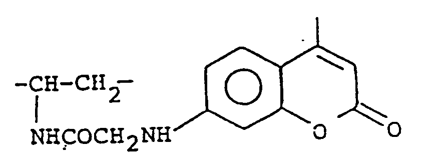 ist, Tu der monoklonale Antikörper Lym 2 ist, Y -CHO ist, Z -CONHNHCH₂- ist und m 0,1 bis 10 ist.

## Revendications (Revendications pour l'(les) Etat(s) contractant(s) suivant(s) : suivants : BE, CH, DE, FR, GB, IT, LI, LU, SE)

1. Conjugué support-médicament de formule 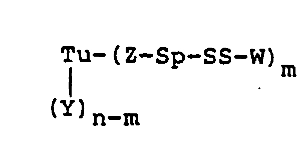 préparé à partir d'un composé de formule CH₃SSS-W, dans laquelle CH₃SSS-W est un antibiotique antitumoral appelé LL-E33288α₁, ^{Br}, α ^{I},α₂^{Br}, α₂l, α₃^{Br}, α₃^{l}, α₄^{Br}, β₁ ^{Br}, β₁, β₂^{Br}, β₂^{l}, β₁ ^{Br}, γ₁ δ₁l, BBM-1675, FR-900405, FR-900406, PD 114759, PD 115028, CL-1577A, CL-1577B, CL-1577D, CL-1577E ou CL-1724 comprenant :
la réaction de CH₃SSS-W avec un composé de formule générale Q-Sp-SH, dans laquelle Sp est un radical divalent (C₁-C₁₈) à chaîne droite ou ramifiée, un radical divalent aryle ou hétéroaryle, un radical divalent cycloalkyle ou hétérocycloalkyle (C₃-C₁₈), un radical divalent aryl- ou hétéroaryl-alkyle (Ci-C₁₈), un radical divalent cycloalkyl- ou hétérocycloalkyl-alkyle (C₁-C₁₈) ou un radical divalent alkyle insaturé (C₂-C₁₈) et Q est ou peut ultérieurement être transformé en un radical halogéno, amino, alkylamino, carboxyle, carboxaldéhyde, hydroxy, thiol, a-halogénoacétyloxy, alkyldicarboxyle inférieur, -CONHNH₂, -NHCONHNH₂, -NHCSNHNH₂, -ONH₂, -CON3, 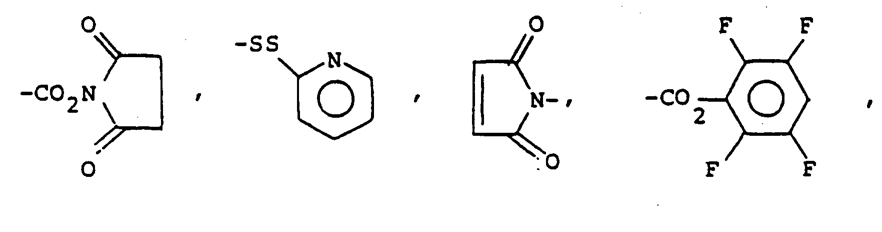 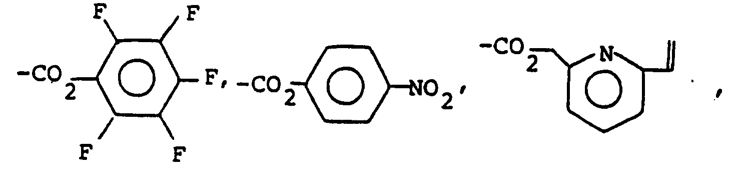 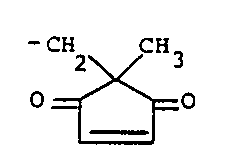 pour produire un intermédiaire de formule Q-Sp-SS-W, dans laquelle Q, Sp et W sont définis comme précédemment,
la réaction de Q-Sp-SS-W avec une molécule de formule Tu-(Y)ₙ, dans laquelle Tu est défini comme un anticorps mono- ou polyclonal, ses fragments ou ses équivalents obtenus par modification chimique ou génétique, des facteurs de croissance ou des stéroïdes ; Y est une chaîne latérale amino, carboxy ou thiol, d'une protéine, un aldéhyde dérivé de restes glucidiques ou un groupe amidoalkylthio ; et n est un entier de 1 à 100, pour produire un composé de formule : 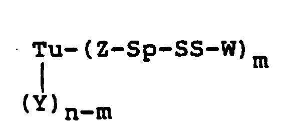 dans laquelle Tu, Y, Sp, W et n sont définis comme précédemment et Z est formé par réaction covalente des groupes Q et Y, directement ou après réduction, et Z est -CONH-, -CONHN=CH-, -CONHNHCH₂-, -NHCONHN=CH-, -NHCONHNHCH₂-, -NHCSNHN=CH-, -NHCSNHNHCH₂-, -ON=CH-, -NH-, -NHCH₂-, -N=CH-, -C0₂-, -NHCH₂C0₂-, -SS-, 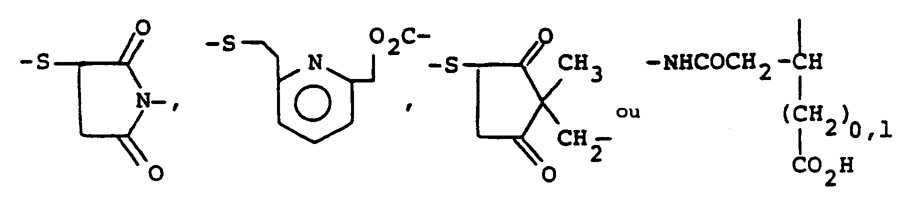 et m a pour valeur 0,1 à 15.

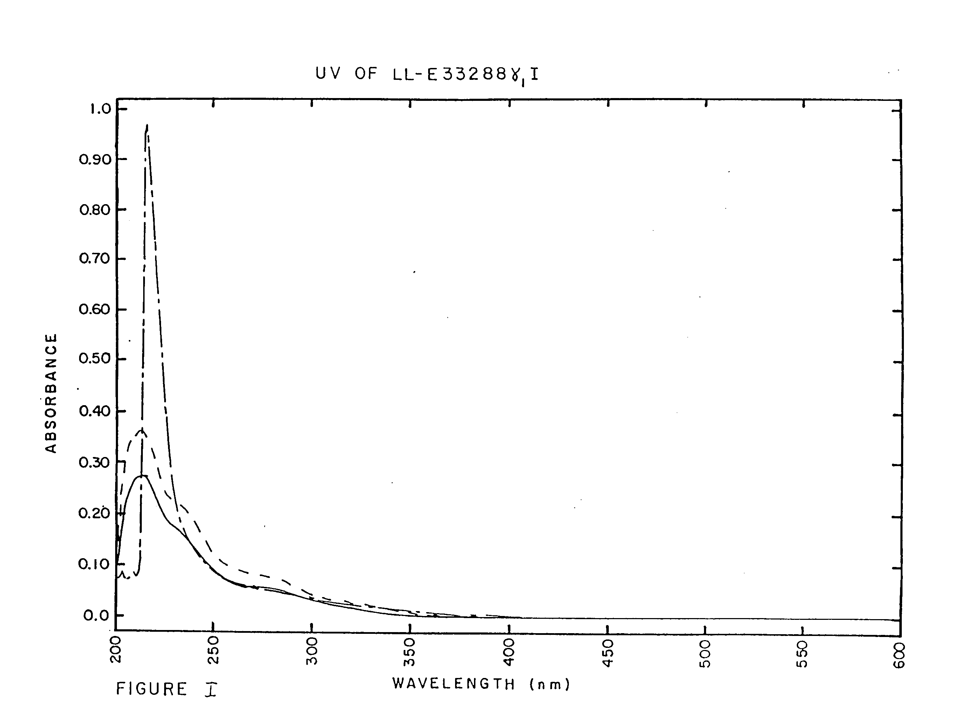

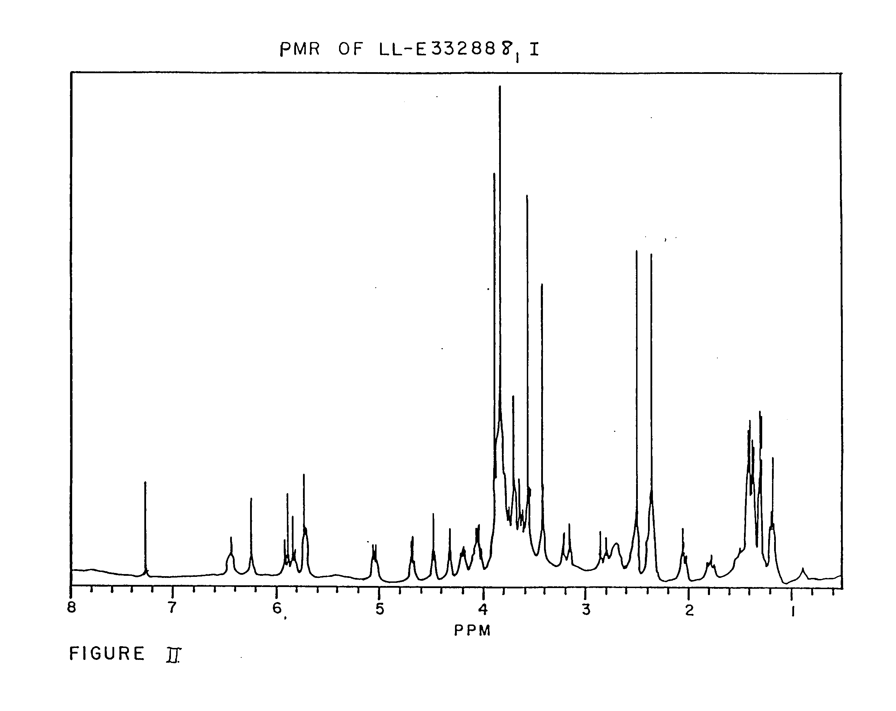

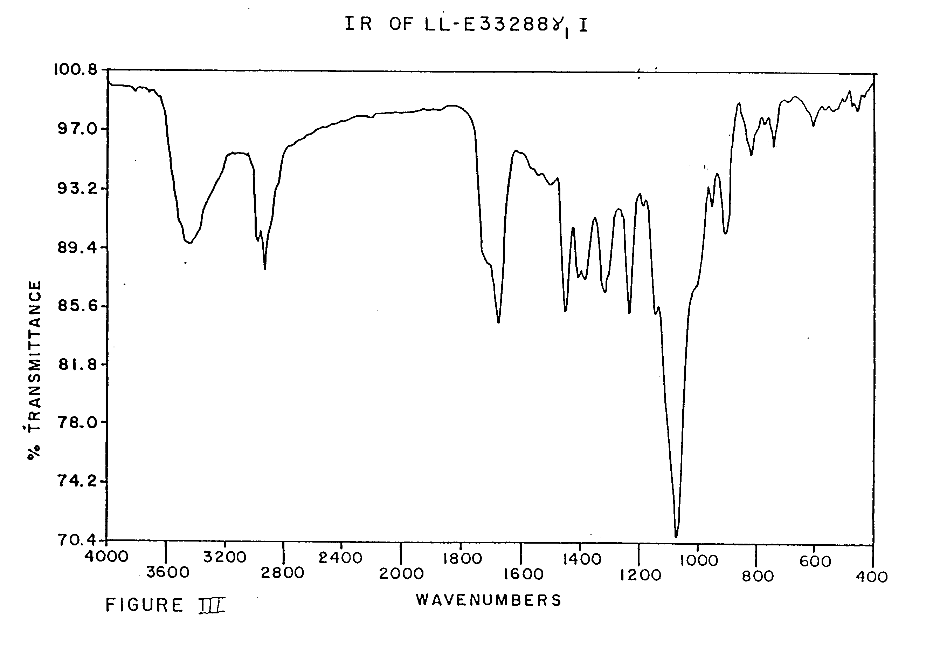

2. Conjugué protéine-médicament de formule 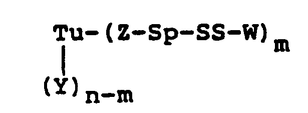 préparé à partir de l'antibiotique antitumoral appelé LL-E33288γ₁l (CH3SSS-W) ayant
a) le spectre ultraviolet illustré par la figure 1 ;
b) un spectre de résonance magnétique protonique illustré par la figure Il ; et
c) un spectre infrarouge illustré par la figure III ;
par :
le déplacement du fragment dithiométhyle avec un composé de formule Q-Sp-SH, dans laquelle Sp est un radical divalent (C₂-Cₛ) à chaîne droite ou ramifiée ou un radical divalent aryl- ou hétéroaryl-alkyle (C₂-C₅) et Q est ou peut ultérieurement être transformé en un groupe carboxyle, anhydride alkyldicarboxylique inférieur, -CONHNH₂ ou 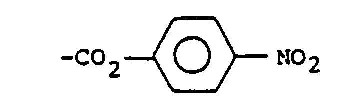 pour produire un intermédiaire de formule générale Q-Sp-SS-W, dans laquelle Q, Sp et W sont définis comme précédemment,
la réaction de Q-Sp-SS-W avec une molécule de formule Tu-(Y)ₙ dans laquelle Tu est un anticorps monoclonal qui présente une réactivité préférentielle avec un antigène tumoral humain, Y est un groupe amino de chaîne latérale sur l'anticorps ou un aldéhyde produit par oxydation des groupes glucidiques de l'anticorps et n est un entier de 1 à 100, pour produire un composé de formule : 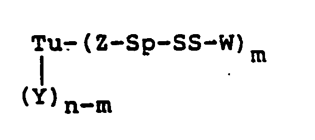 dans laquelle Tu, Y, Sp, W et n sont définis comme précédemment et Z est formé par la réaction covalente des groupes Q et Y directement ou après réduction, et Z est -CONH-, -CONHN=CH-, -CONHNHCH₂- ou 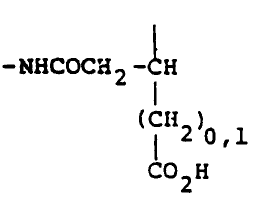 et m a pour valeur 0,1 à 15.

3. Composé selon la revendication 1, où CH₃SSS-W est LL-E33288γ₁^{l}, Q est l'ester 4-nitrophénylique d'un groupe carboxyle, Sp est -CH₂CH₂-, Tu est l'anticorps monoclonal CT-M-01, Y est -NH₂, Z est -CONH- et m a pour valeur 0,5 à 15.

4. Composé selon la revendication 1, où CH₃SSS-W est LL-E33288γ₁^{l}, Q est l'ester hydroxysuccinimidique d'un groupe carboxyle, Sp est -CH₂CH₂-, Tu est l'anticorps monoclonal MAC-68, Y est -NH₂, Z est -CONH- et m a pour valeur 0,5 à 15.

5. Composé selon la revendication 1 où CH₃SSS-W est LL-E33288_{γ1}l, q est -CONHNH₂, Sp est -CH₂CH₂-, Tu est l'anticorps monoclonal Lym 1, Y est -CHO, Z est -CONHNHCH₂- et m a pour valeur 0,1 à 10.

6. Composé selon la revendication 1, où CH₃SSS-W est LL-E33288_{γ1}^{l}, Sp est 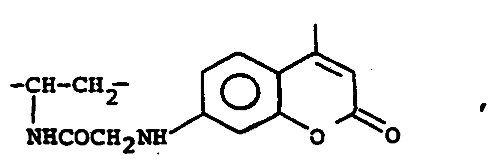 Tu est l'anticorps monoclonal B72.3, Y est -CHO, Z est -CONHNHCH₂- et m a pour valeur 0,1 à 10.

7. Composé selon la revendication 1, où CH₃SSS-W est LL-E33288γ₁^{l}, Sp est 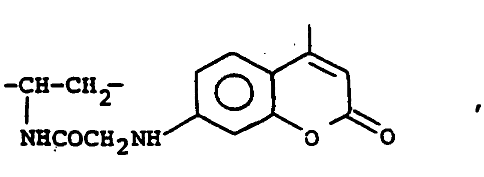 Tu est l'anticorps monoclonal Lym 2, Y est -CHO, Z est -CONHNHCH₂- et m a pour valeur 0,1 à 10.

8. Procédé pour préparer les dérivés dirigés vers une cible 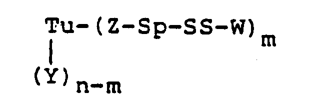 de composés de formule CH₃SSS-W, dans laquelle CH₃SSS-W est un antibiotique antitumoral LL-E33288ₐₗ ^{Br}, α₁^{l}, α₂^{Br}, α₂^{l}, α₃^{Br}, α₃^{l}, α₄^{Br}, β₁ ^{Br}, β₁^{l}, β₂^{Br}, β₂^{l}, γ₁^{Br}, γ₁^{l}, δ₁^{l}, BBM-1675, FR-900405, FR-900406, PD 114759, PD 115028, CL-1577A, CL-1577B, CL-1577D, CL-1577E ou CL-1724 comprenant :
la réaction de CH₃SSS-W avec un composé de formule générale Q-Sp-SH, dans laquelle Sp est un radical divalent (C₁-C₁₈) à chaîne droite ou ramifiée, un radical divalent aryle ou hétéroaryle, un radical divalent cycloalkyle ou hétérocycloalkyle (C₃-C₁₈), un radical divalent aryl- ou hétéroaryl-alkyle (Ci-C₁₈), un radical divalent cycloalkyl- ou hétérocycloalkyl-alkyle (C₁-C₁₈) ou un radical divalent alkyle insaturé (C₂-Cₗ₈) et Q est un radical halogéno, amino, alkylamino, carboxyle, carboxaldéhyde, hydroxy, anhydride alkyldicarboxylique inférieur, -CONHNH₂, -NHCONHNH₂, -NHCSNHNH₂, -ONH₂ ou 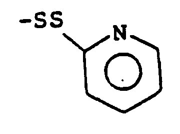 dans l'acétonitrile en présence d'un équivalent de triéthylamine et/ou d'un équivalent d'acide acétique entre -10 ° et -30 °C pendant 1 à 48 heures,
l'isolement de l'intermédiaire de formule Q-Sp-SS-W, dans laquelle Q, Sp et W sont définis comme précédemment, puis
la réaction du composé de formule Q-Sp-SS-W, dans laquelle Sp et W sont définis comme précédemment et Q est un radical halogéno, amino, alkylamino, carboxyle, carboxaldéhyde, hydroxy ou anhydride alkyldicarboxylique inférieur, avec une molécule de formule Tu-(Y)ₙ dans laquelle Tu est un anticorps mono- ou polyclonal, ses fragments ou ses équivalents obtenus par modification chimique ou génétique, des facteurs de croissance ou des stéroïdes ; Y est une fonction amino ou carboxy en chaîne latérale ; n est 1 à 100, dans un tampon aqueux à un pH entre 6,5 et 9, entre 4 ° et 40 _{°} C, soit directement soit en présence d'un carbodiimide soluble dans l'eau, pour produire le composé 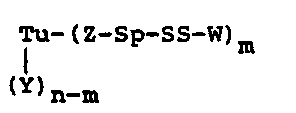 dans laquelle Tu, Sp, W, n et Y sont définis comme précédemment, m a pour valeur 1 à 15 et Z est formé à partir d'une réaction covalente des groupes Q et Y et est -CONH-, -NH-, 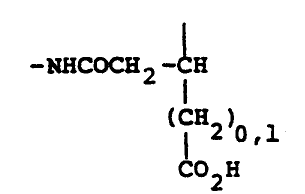 -N = CH- ou -C0₂-ou
la réaction du composé de formule Q-Sp-SS-W, dans laquelle Sp et W sont définis comme précédemment et Q est un acide carboxylique, avec le N-hydroxysuccinimide, le 2,3,5,6-tétrafluorophé- nol, le pentafluorophénol ou le 4-nitrophénol, en présence d'un agent d'activation de la fonction carboxyle, tel qu'un carbodiimide, pour produire un composé de formule Q-Sp-SS-W, dans laquelle Sp et W sont comme précédemment défini et Q est 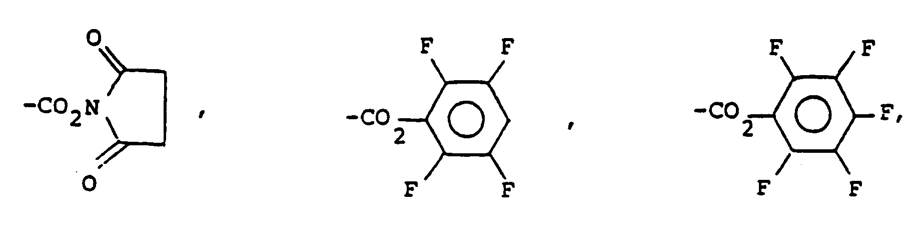 ou 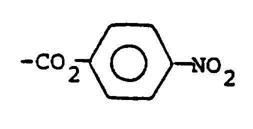 avec une molécule de formule Tu-(Y)ₙ,
dans laquelle Tu et n sont définis comme précédemment et Y est une chaîne latérale amino , dans une solution aqueuse tamponnée à un pH entre 6,5 et 9, à une température entre 4° et 40°C inclusivement, pour former des composés de formule : 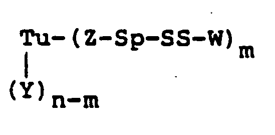 dans laquelle Tu, Sp, Y et n sont définis comme précédemment, m a une valeur de 1 à 15 et Z est formé par réaction covalente entre Q et Y et est défini comme -CONH-ou
la réaction d'un composé de formule Q-Sp-SS-W, dans laquelle Sp et W sont définis comme précédemment et Q est -CONHNH₂, avec l'acide nitreux dans l'acétonitrile aqueux, pour former un composé de formule Q-Sp-SS-W, dans laquelle Sp et W sont définis comme précédemment et Q est -CON3, avec un composé de formule Tu-(Y)ₙ, dans laquelle Tu, Y et n sont définis comme ci-dessus, pour produire un composé de formule 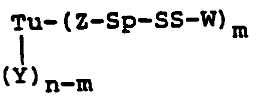 dans laquelle Tu, Z, Sp, W, m, Y et n sont définis comme ci-dessus, ou
la réaction d'un composé de formule Q-Sp-SS-W, dans laquelle Sp et W sont définis comme précédemment et Q est un radical hydroxy, avec un anhydride a-halogénoacétique, pour produire un composé dans lequel Q est un radical a-halogénoacétyloxy, et la réaction de l'a-halogénoacétyloxy-Sp-SS-W ou d'un composé de formule Q-Sp-SS-W, où Sp et W sont définis comme précédemment et Q est 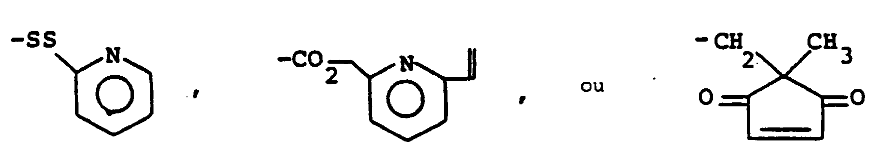 avec une molécule de formule Tu-(Y)ₙ, dans laquelle Tu est défini comme précédemment, Y est une chaîne latérale thiol d'une protéine, ou un groupe amidoalkylthio introduit sur une amine de Tu par utilisation de composés réagissants tels que l'ester hydroxysuccinimidique de l'acide 3-(2-dithiopyri- dyl)propionique, puis réduction avec un agent tel que le dithiothréitol, ou un groupe amidoalkylthio introduit sur une amine de Tu par utilisation de 2-aminothiolanne, et n a une valeur de 1 à 10, dans des conditions tamponnées aqueuses, à un pH entre 4,5 et 7, à une température entre 4° et 40 °C inclusivement, pour produire un composé de formule : 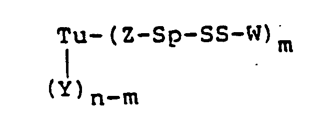 dans laquelle Tu, Sp, W et n sont définis comme précédemment et Z est formé par réaction covalente des groupes Q et Y, et Z est 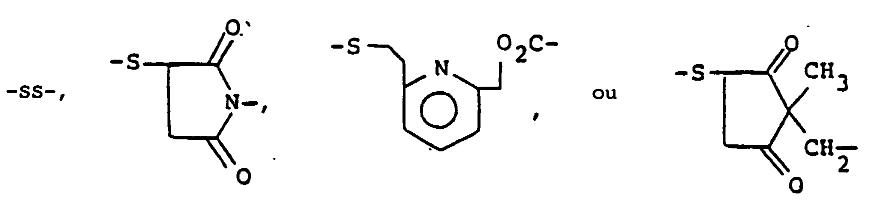 et n a une valeur de 0,1 à 10 ; ou
la réaction d'un composé de formule Q-Sp-SS-W, dans laquelle Sp et W sont définis comme précédemment et Q est -NH₂, -CONHNH₂, -NHCONHNH₂, -NHCSNHNH₂ ou -ONH₂, avec une molécule de formule Tu-(Y)ₙ, dans laquelle Tu est défini comme précédemment, Y est un aldéhyde formé à partir de restes glucidiques sur Tu par oxydation en présence d'un periodate de métal alcalino- terreux, dans un tampon aqueux à un pH entre 4,0 et 6,5, entre 4 ° et 40 °C inclusivement, et n a une valeur de 1 à 20, pour produire un composé de formule : 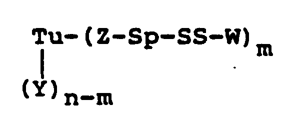 dans laquelle Tu, Sp, W, Y et n sont définis comme précédemment et Z est formé par réaction covalente de Q et Y et est -ON=CH-, -N=CH-, -CONHN=CH-, -NHCONHN=CH- ou -NHCSNHN=CH- et m a une valeur de 0,1 à 15 ; ou le traitement du composé immédiatement précédent de formule : 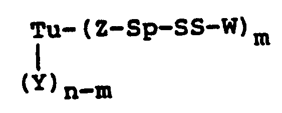 dans laquelle Tu, Z, Sp, W, Y, n et m sont comme immédiatement définis ci-dessus, avec l'acétylhy- drazine ou la tyrosine-hydrazine, dans un tampon aqueux à un pH entre 4,0 et 6,5, entre 4 et 40 °C inclusivement, pour produire un composé de formule : 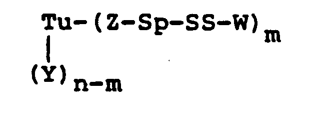 dans laquelle Tu, Z, Sp, W, n et m sont comme immédiatement définis ci-dessus et Y est -CH = NNHCOCH₃ ou 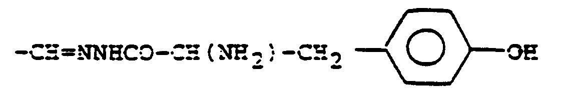 et
la réaction de ce composé avec le cyanoborohydrure de sodium ou le borohydrure de sodium, dans un tampon aqueux à un pH de 4,0 à 6,5, à une température de 4 à à 40 °C inclusivement, pour produire un composé de formule : 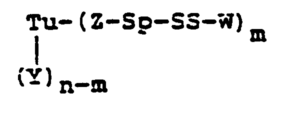 dans laquelle Tu, Sp, W, m et n sont définis comme ci-dessus, Z est -NH-CH₂-, -CONHNHCH₂-, -NHCONHNHCH₂- ou -NHCSNHNHCH₂-, et Y est -CH₂NHNHCOCH₃ ou

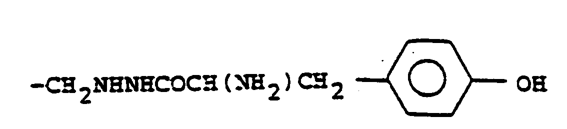

9. Composition pharmaceutique contenant un conjugué support-médicament selon l'une quelconque des revendications 1 à 7.

10. Utilisation d'un conjugué support-médicament selon l'une quelconque des revendications 1 à 7, pour la préparation d'un médicament pour inhiber la croissance des tumeurs chez un mammifère.

## Revendications (Revendications pour l'(les) Etat(s) contractant(s) suivant(s) : suivants : ES, GR)

1. Procédé pour la préparation de dérivés, dirigés vers une cible : 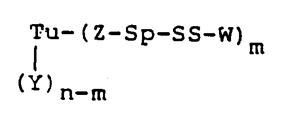 de composés de formule CH₃SSS-W, dans laquelle CH₃SSS-W est un antibiotique antitumoral LL-E33288α1 ^{Br}, α₁l, α₂Br, α₂^{l}, α₃^{Br}, α₃^{l}, α₄^{Br}, β₁ ^{Br}, β₁^{l} β₂^{Br}, β₂^{l}, γ₁ ^{Br}, γ₁^{l}, δ₁^{l}, BBM-1675, FR-900405, FR-900406, PD 114759, PD 115028, CL-1577A, CL-1577B, CL-1577D, CL-1577E ou CL-1724 comprenant :
la réaction de CH₃SSS-W avec un composé de formule générale Q-Sp-SH, dans laquelle Sp est un radical divalent (C₁-C₁₈) à chaîne droite ou ramifiée, un radical divalent aryle ou hétéroaryle, un radical divalent cycloalkyle ou hétérocycloalkyle (C₃-C₁₈), un radical divalent aryl- ou hétéroaryl-alkyle (Ci-C₁₈), un radical divalent cycloalkyl- ou hétérocycloalkyl-alkyle (C₁-C₁₈) ou un radical divalent alkyle insaturé (C₂-Cₗ₈) et Q est un radical halogéno, amino, alkylamino, carboxyle, carboxaldéhyde, hydroxy, anhydride alkyldicarboxylique inférieur, -CONHNH₂, -NHCONHNH₂, -NHCSNHNH₂, -ONH₂ ou 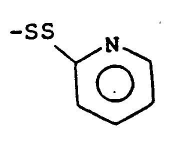 dans l'acétonitrile en présence d'un équivalent de triéthylamine et/ou d'un équivalent d'acide acétique entre -10 ° et -30 °C pendant 1 à 48 heures,
l'isolement de l'intermédiaire de formule Q-Sp-SS-W, dans laquelle Q, Sp et W sont définis comme précédemment, puis
la réaction du composé de formule Q-Sp-SS-W, dans laquelle Sp et W sont définis comme précédemment et Q est un radical halogéno, amino, alkylamino, carboxyle, carboxaldéhyde, hydroxy ou anhydride alkyldicarboxylique inférieur, avec une molécule de formule Tu-(Y)ₙ dans laquelle Tu est un anticorps mono- ou polyclonal, ses fragments ou ses équivalents obtenus par modification chimique ou génétique, des facteurs de croissance ou des stéroïdes ; Y est une fonction amino ou carboxy en chaîne latérale ; n est 1 à 100, dans un tampon aqueux à un pH entre 6,5 et 9, entre 4 ° et 40 °C, soit directement soit en présence d'un carbodiimide soluble dans l'eau, pour produire le composé 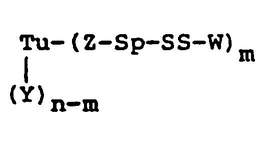 dans laquelle Tu, Sp, W, n et Y sont définis comme précédemment, m a pour valeur 1 à 15 et Z est formé à partir d'une réaction covalente des groupes Q et Y et est -CONH-, -NH-, 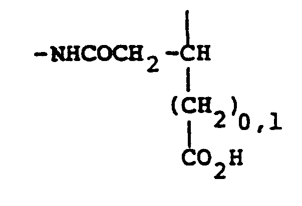 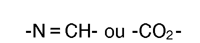 ou
la réaction du composé de formule Q-Sp-SS-W, dans laquelle Sp et W sont définis comme précédemment et Q est un acide carboxylique, avec le N-hydroxysuccinimide, le 2,3,5,6-tétrafluorophé- nol, le pentafluorophénol ou le 4-nitrophénol, en présence d'un agent d'activation de la fonction carboxyle, tel qu'un carbodiimide, pour produire un composé de formule Q-Sp-SS-W, dans laquelle Sp et W sont comme précédemment défini et Q est 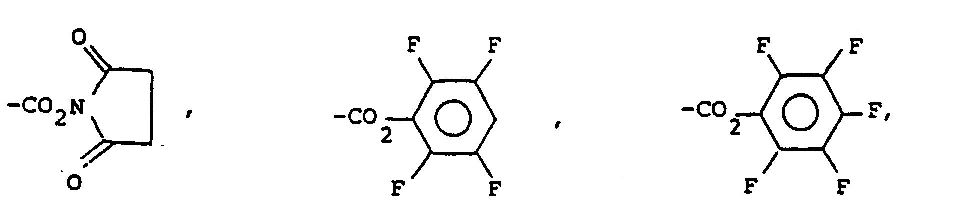 ou 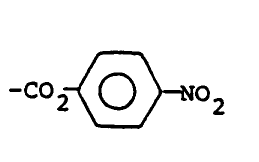 avec une molécule de formule Tu-(Y)ₙ,
dans laquelle Tu et n sont définis comme précédemment et Y est une chaîne latérale amino , dans une solution aqueuse tamponnée à un pH entre 6,5 et 9, à une température entre 4° et 40°C inclusivement, pour former des composés de formule : 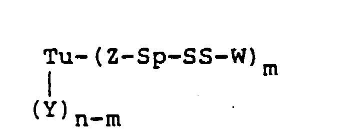 dans laquelle Tu, Sp, Y et n sont définis comme précédemment, m a une valeur de 1 à 15 et Z est formé par réaction covalente entre Q et Y et est défini comme -CONH-ou
la réaction d'un composé de formule Q-Sp-SS-W, dans laquelle Sp et W sont définis comme précédemment et Q est -CONHNH₂, avec l'acide nitreux dans l'acétonitrile aqueux, pour former un composé de formule Q-Sp-SS-W, dans laquelle Sp et W sont définis comme précédemment et Q est -CON3, avec un composé de formule Tu-(Y)ₙ, dans laquelle Tu, Y et n sont définis comme ci-dessus, pour produire un composé de formule 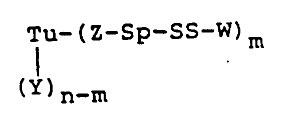 dans laquelle Tu, Z, Sp, W, m, Y et n sont définis comme ci-dessus, ou
la réaction d'un composé de formule Q-Sp-SS-W, dans laquelle Sp et W sont définis comme précédemment et Q est un radical hydroxy, avec un anhydride a-halogénoacétique, pour produire un composé dans lequel Q est un radical a-halogénoacétyloxy, et la réaction de l'a-halogénoacétyloxy-Sp-SS-W ou d'un composé de formule Q-Sp-SS-W, où Sp et W sont définis comme précédemment et Q est 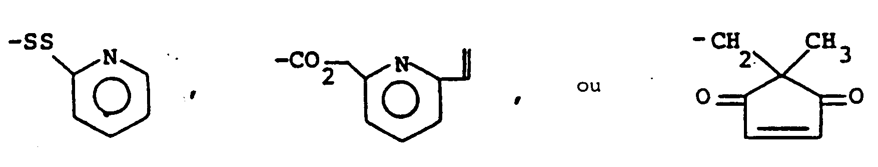 avec une molécule de formule Tu-(Y)ₙ, dans laquelle Tu est défini comme précédemment, Y est une chaîne latérale thiol d'une protéine, ou un groupe amidoalkylthio introduit sur une amine de Tu par utilisation de composés réagissants tels que l'ester hydroxysuccinimidique de l'acide 3-(2-dithiopyri- dyl)propionique, puis réduction avec un agent tel que le dithiothréitol, ou un groupe amidoalkylthio introduit sur une amine de Tu par utilisation de 2-aminothiolanne, et n a une valeur de 1 à 10, dans des conditions tamponnées aqueuses, à un pH entre 4,5 et 7, à une température entre 4° et 40 °C inclusivement, pour produire un composé de formule : 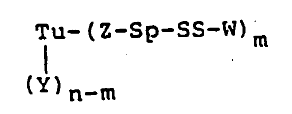 dans laquelle Tu, Sp, W et n sont définis comme précédemment et Z est formé par réaction covalente des groupes Q et Y, et Z est 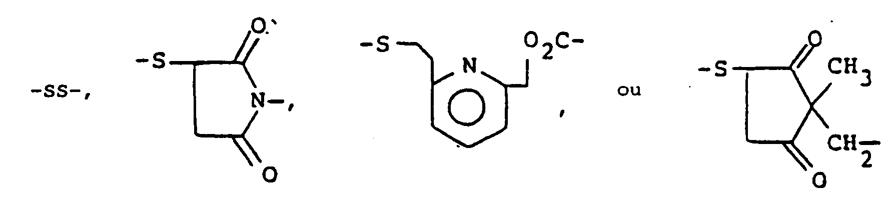 et n a une valeur de 0,1 à 10 ; ou
la réaction d'un composé de formule Q-Sp-SS-W, dans laquelle Sp et W sont définis comme précédemment et Q est -NH₂, -CONHNH₂, -NHCONHNH₂, -NHCSNHNH₂ ou -ONH₂, avec une molécule de formule Tu-(Y)ₙ, dans laquelle Tu est défini comme précédemment, Y est un aldéhyde formé à partir de restes glucidiques sur Tu par oxydation en présence d'un periodate de métal alcalino- terreux, dans un tampon aqueux à un pH entre 4,0 et 6,5, entre 4 ° et 40 °C inclusivement, et n a une valeur de 1 à 20, pour produire un composé de formule : 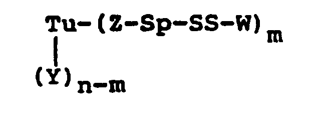 dans laquelle Tu, Sp, W, Y et n sont définis comme précédemment et Z est formé par réaction covalente de Q et Y et est -ON=CH-, -N=CH-, -CONHN=CH-, -NHCONHN=CH- ou -NHCSNHN=CH- et m a une valeur de 0,1 à 15 ; ou le traitement du composé immédiatement précédent de formule : 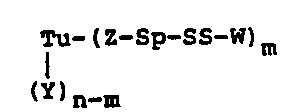 dans laquelle Tu, Z, Sp, W, Y, n et m sont comme immédiatement définis ci-dessus, avec l'acétylhy- drazine ou la tyrosine-hydrazine, dans un tampon aqueux à un pH entre 4,0 et 6,5, entre 4 et 40 °C inclusivement, pour produire un composé de formule : 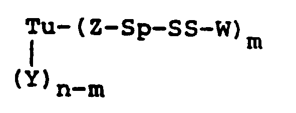 dans laquelle Tu, Z, Sp, W, n et m sont comme immédiatement définis ci-dessus et Y est -CH = NNHCOCH₃ ou 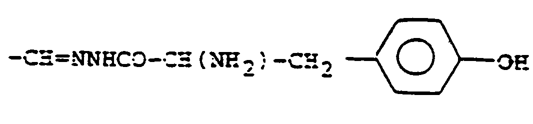 et
la réaction de ce composé avec le cyanoborohydrure de sodium ou le borohydrure de sodium, dans un tampon aqueux à un pH de 4,0 à 6,5, à une température de 4 à à 40 °C inclusivement, pour produire un composé de formule : 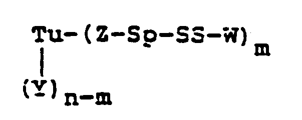 dans laquelle Tu, Sp, W, m et n sont définis comme ci-dessus, Z est -NH-CH₂-, -CONHNHCH₂-, -NHCONHNHCH₂- ou -NHCSNHNHCH₂-, et Y est -CH₂NHNHCOCH₃ ou

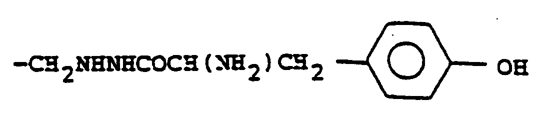

Procédé selon la revendication 1 pour la préparation d'un conjugué support-médicament de formule: 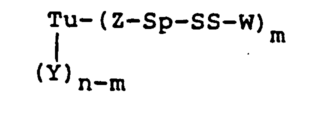 préparé à partir d'un composé de formule CH₃SSS-W, dans laquelle CH₃SSS-W est un antibiotique antitumoral appelé LL-E33288α₁^{Br}, α₁,_{α2}^{Br}, α₂^{l}, α₃^{Br}, α₃^{l}, α₄^{Br}, β₁ ^{Br}, β₁ β₂, ^{Br}, β₂^{l}, γ₁ Bᵣ, γ₁ δ₁^{l}, BBM-1675, FR-900405, FR-900406, PD 114759, PD 115028, CL-1577A, CL-1577B, CL-1577D, CL-1577E ou CL-1724 comprenant :
la réaction de CH₃SSS-W avec un composé de formule générale Q-Sp-SH, dans laquelle Sp est un radical divalent (C₁-C₁₈) à chaîne droite ou ramifiée, un radical divalent aryle ou hétéroaryle, un radical divalent cycloalkyle ou hétérocycloalkyle (C₃-C₁₈), un radical divalent aryl- ou hétéroaryl-alkyle (Ci-C₁₈), un radical divalent cycloalkyl- ou hétérocycloalkyl-alkyle (C₁-C₁₈) ou un radical divalent alkyle insaturé (C₂-C₁₈) et Q est ou peut ultérieurement être transformé en un radical halogéno, amino, alkylamino, carboxyle, carboxaldéhyde, hydroxy, thiol, a-halogénoacétyloxy, alkyldicarboxyle inférieur, -CONHNH₂, -NHCONHNH₂, -NHCSNHNH₂, -ONH₂, -CON3, 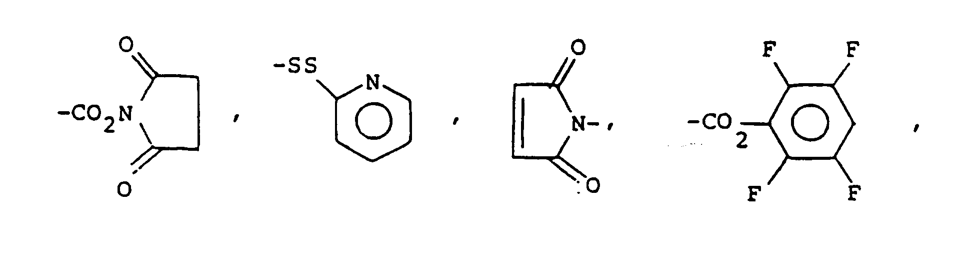 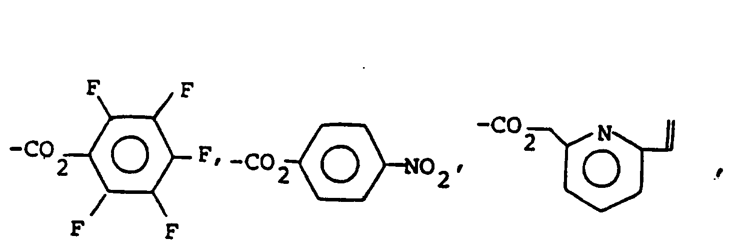 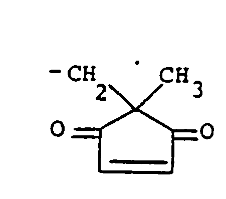 pour produire un intermédiaire de formule Q-Sp-SS-W, dans laquelle Q, Sp et W sont définis comme précédemment,
la réaction de Q-Sp-SS-W avec une molécule de formule Tu-(Y)ₙ, dans laquelle Tu est défini comme un anticorps mono- ou polyclonal, ses fragments ou ses équivalents obtenus par modification chimique ou génétique, des facteurs de croissance ou des stéroïdes ; Y est une chaîne latérale amino, carboxy ou thiol, d'une protéine, un aldéhyde dérivé de restes glucidiques ou un groupe amidoalkylthio ; et n est un entier de 1 à 100, pour produire un composé de formule : 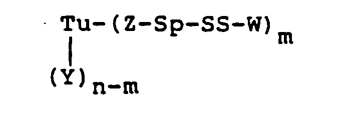 dans laquelle Tu, Y, Sp, W et n sont définis comme précédemment et Z est formé par réaction covalente des groupes Q et Y, directement ou après réduction, et Z est -CONH-, -CONHN=CH-, -CONHNHCH₂-, -NHCONHN=CH-, -NHCONHNHCH₂-, -NHCSNHN=CH-, -NHCSNHNHCH₂-, -ON=CH-, -NH-, -NHCH₂-, -N=CH-, -C0₂-, -NHCH₂C0₂-, -SS-, 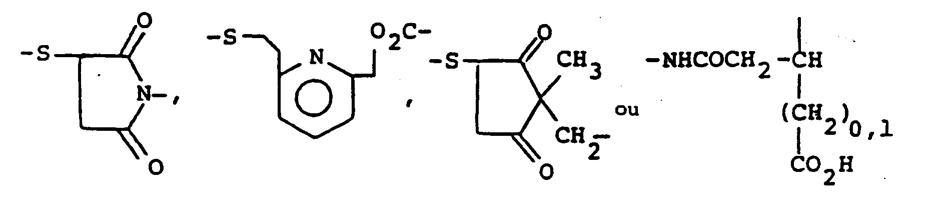 et m a pour valeur 0,1 à 15.

3. Procédé selon la revendication 1 pour la préparation d'un conjugué protéine-médicament 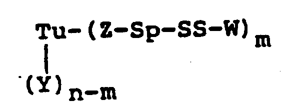 préparé à partir de l'antibiotique antitumoral appelé LL-E33288γ₁l (CH3SSS-W) ayant
a) le spectre ultraviolet illustré par la figure 1 ;
b) un spectre de résonance magnétique protonique illustré par la figure Il ; et
c) un spectre infrarouge illustré par la figure III ;
par :
le déplacement du fragment dithiométhyle avec un composé de formule Q-Sp-SH, dans laquelle Sp est un radical divalent (C₂-Cₛ) à chaîne droite ou ramifiée ou un radical divalent aryl- ou hétéroaryl-alkyle (C₂-C₅) et Q est ou peut ultérieurement être transformé en un groupe carboxyle, anhydride alkyldicarboxylique inférieur, -CONHNH₂ ou 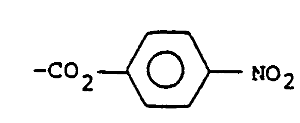 pour produire un intermédiaire de formule générale Q-Sp-SS-W, dans laquelle Q, Sp et W sont définis comme précédemment,
la réaction de Q-Sp-SS-W avec une molécule de formule Tu-(Y)ₙ dans laquelle Tu est un anticorps monoclonal qui présente une réactivité préférentielle avec un antigène tumoral humain, Y est un groupe amino de chaîne latérale sur l'anticorps ou un aldéhyde produit par oxydation des groupes glucidiques de l'anticorps et n est un entier de 1 à 100, pour produire un composé de formule : 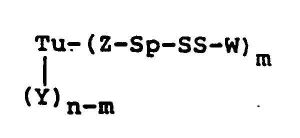 dans laquelle Tu, Y, Sp, W et n sont définis comme précédemment et Z est formé par la réaction covalente des groupes Q et Y directement ou après réduction, et Z est -CONH-, -CONHN=CH-, -CONHNHCH₂- ou 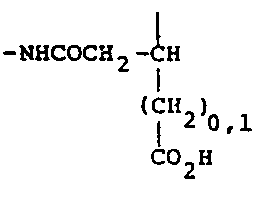 et m a pour valeur 0,1 à 15.

4. Procédé selon la revendication 1, où CH₃SSS-W est LL-E33288γ₁^{l}, Q est l'ester 4-nitrophénylique d'un groupe carboxyle, Sp est -CH₂CH₂-, Tu est l'anticorps monoclonal CT-M-01, Y est -NH₂, Z est -CONH-et m a pour valeur 0,5 à 15.

5. Procédé selon la revendication 1, où CH₃SSS-W est LL-E33288_{γ1}^{l}, Q est l'ester hydroxysuccinimidique d'un groupe carboxyle, Sp est -CH₂CH₂-, Tu est l'anticorps monoclonal MAC-68, Y est -NH₂, Z est -CONH- et m a pour valeur 0,5 à 15.

6. Procédé selon la revendication 1 où CH₃SSS-W est LL-E33288_{γ1} q est -CONHNH₂, Sp est -CH₂CH₂-, Tu est l'anticorps monoclonal Lym 1, Y est -CHO, Z est -CONHNHCH₂- et m a pour valeur 0,1 à 10.

7. Procédé selon la revendication 1, où CH₃SSS-W est LL-E33288_{γ1}^{l}, Sp est 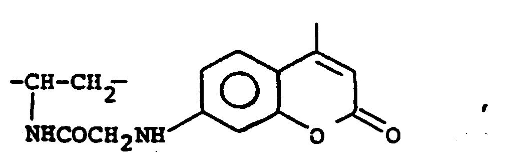 Tu est l'anticorps monoclonal B72.3, Y est -CHO, Z est -CONHNHCH₂- et m a pour valeur 0,1 à 10.

8. Procédé selon la revendication 1, où CH₃SSS-W est LL-E33288γ₁^{l}, Sp est 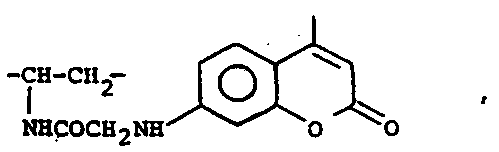 Tu est l'anticorps monoclonal Lym 2, Y est -CHO, Z est -CONHNHCH₂- et m a pour valeur 0,1 à 10.
